# EUROPÄISCHE PATENTANMELDUNG

(11) **EP 1 764 109 A1**
(43) Veröffentlichungstag der Anmeldung: **21.03.2007**
(21) Anmeldenummer: 05011275.4
(22) Anmeldetag: 24.05.2005
(51) Int. Cl.: A61K 39/395, C07K 14/705, C07K 16/28, A61K 39/00, A61P 3/04, G01N 33/50, A61K 48/00

(54) **Agonistische Antikörper, welche an den Tweak Rezeptor Fn14 binden und dadurch Adipositas assoziierte Phänotypen modulieren, und deren Verwendung in der Therapie**

(71) Anmelder: Xantos Biomedicine AG, 81377 München (DE)
(72) Erfinder: Link, Dieter, Dr., 82152 Planegg (DE)

(57) **Zusammenfassung**

Die vorliegende Erfindung betrifft ein Fn14-aktivierendes Agens zur Vorbeugung bzw. Behandlung von Adipositas-assozüerter Diabetes (Typ II) und/oder Metabolischem Syndrom und/oder anderen Adipositas-assoziierten Krankheiten.

SEQUENCE LISTING

<110>  Xantos Biomedicine AG

<120>  Agonistische Antikörper, welche an den Tweak Rezeptor Fn14 binden
        und dadurch Adipositas assoziierte Phänotypen modulieren, und
       deren Verwendung in der Therapie

<130>  EP34999

<160>  4

<170>  PatentIn version 3.3

<210>  1
<211>  40
<212>  DNA
<213>  Artificial

<220>
<223>  flankierender vorwärts Primer für die lösliche, aktive Domäne von
       murinem TNFSF12

<400>  1
catcaccatc accatcaccg agctattgca gcccattatg                              40


<210>  2
<211>  30
<212>  DNA
<213>  Artificial

<220>
<223>  flankierender rückwärts Primer für die lösliche, aktive Domäne
       von murinem TNFSF12

<400>  2
agatctcgag tcatcagtga acttgaaaga                                         30


<210>  3
<211>  187
<212>  PRT
<213>  Mus musculus

<400>  3

Met Glu Thr Asp Thr Leu Leu Leu Trp Val Leu Leu Leu Trp Val Pro
1               5                   10                  15


Gly Ser Thr Gly Asp Ala Ala Gln Pro Ala Arg Arg Ala Arg Arg Thr
            20                  25                  30


Lys Leu Ala Leu His His His His His His Arg Ala Ile Ala Ala His
        35                  40                  45


Tyr Glu Val His Pro Arg Pro Gly Gln Asp Gly Ala Gln Ala Gly Val
    50                  55                  60


Asp Gly Thr Val Ser Gly Trp Glu Glu Thr Lys Ile Asn Ser Ser Ser
65                  70                  75                  80


Pro Leu Arg Tyr Asp Arg Gln Ile Gly Glu Phe Thr Val Ile Arg Ala
                85                  90                  95

Gly Leu Tyr Tyr Leu Tyr Cys Gln Val His Phe Asp Glu Gly Lys Ala
            100                 105                 110

Val Tyr Leu Lys Leu Asp Leu Leu Val Asn Gly Val Leu Ala Leu Arg
        115                 120                 125

Cys Leu Glu Glu Phe Ser Ala Thr Ala Ala Ser Ser Pro Gly Pro Gln
    130                 135                 140

Leu Arg Leu Cys Gln Val Ser Gly Leu Leu Pro Leu Arg Pro Gly Ser
145                 150                 155                 160

Ser Leu Arg Ile Arg Thr Leu Pro Trp Ala His Leu Lys Ala Ala Pro
                165                 170                 175

Phe Leu Thr Tyr Phe Gly Leu Phe Gln Val His
            180                 185

<210>  4
<211>  15
<212>  PRT
<213>  Homo sapiens

<400>  4

Met Asp Cys Ala Ser Cys Arg Ala Arg Pro His Ser Asp Phe Cys
1               5                   10                  15

## Beschreibung

### Technische Beschreibung der Erfindung

Die Erfindung betrifft pharmazeutische Zusammensetzungen sowie deren Verwendung zur Aktivierung des Signalwegs des Rezeptors Fn14 (Synonyme sind auch TWEAK-Rezeptor, TWEAK-R oder TNFRSF12A), zur Interferenz und / oder Modulation von Adipozytendifferenzierung und zur Anwendung in Adipositas und/oder Metabolischem Syndrom. Die Erfindung betrifft insbesondere Antikörper, welche durch rezeptorvermittelte Signaltransduktion die Differenzierung von Prä-Adipozyten zu reifen Adipozyten, und/oder die Einlagerung von Lipidmolekülen in differenzierenden oder reifen Adipozyten hemmen. Die Erfindung betrifft ebenfalls definierte Epitope auf dem Rezeptor Fn14, durch deren spezifische Antikörperbindung der Rezeptor zur Signaltransduktion gebracht wird. Insbesonders betrifft die Erfindung Antikörper, die gegen definierte aktivierende Epitope auf dem Rezeptor Fn14 gerichtet sind und die als Fn14-aktivierende Agenzien agieren. Diese Aktivierung kann vorzugsweise allein oder auch in Kombination mit anderen Fn14-aktivierenden Agenzien stattfinden. Weiterhin betrifft die Erfindung pharmazeutische Zusammensetzungen und deren Verwendung zur Aktivierung des Rezeptor Fn14-Signalwegs, zur Interferenz und / oder Modulation von Adipozytendifferenzierung, insbesondere zur Prävention und/oder Behandlung von Adipositas.

### Hintergrund der Erfindung

Adipositas (Fettleibigkeit) und Typ II Diabetes sind zwei der derzeit wichtigsten Stoffwechselerkrankungen, die gerade in der westlichen Welt zunehmend ein ernstes Problem darstellen. Ferner sind gerade auch beim Typ II Diabetes inzwischen Tendenzen zu einer Pandemie zu beobachten.

Adipositas ist ein Zustand, der durch eine übermäßige Ansammlung von Fettgewebe im Körper gekennzeichnet ist. Wesentlich für die Bildung von Fettgewebe und damit zur Ausprägung eines fettleibigen Phänotyps sind die Adipozyten, d.h. diejenigen Zellen, welche das Fettgewebe des Körpers bilden. Nach neuerem Verständnis ist die Adipositas eine chronische Gesundheitsstörung, die sowohl auf einer polygenetischen Veranlagung beruht als auch durch Umweltfaktoren bedingt ist und mit einer hohen Begleit- und Folgemorbidität einhergeht. Hierbei ist Adipositas mit dem signifikanten Risiko, insbesondere der Entwicklung eines Typ II Diabetes verbunden. Daher ist für Adipositas ein langfristiges Behandlungs- und Betreuungskonzept unerlässlich.

Das Vorliegen von Fettleibigkeit, Fettsucht bzw. Adipositas kann als Übergewichtigkeit oder (klinische) Adipositas im engeren Sinne klassifiziert werden. Sie kann über den so genannten "Body Mass Index" (BMI) definiert werden. Der BMI beschreibt das Verhältnis des Körpergewichtes zur Körpergröße. Der BMI wird nach der folgenden Formel berechnet: BMI = Körpergewicht (kg) geteilt durch die Körpergröße zum Quadrat (m²).

Die Klassifizierung des BMI wird von der WHO (Weltgesundheitsorganisation) in Abhängigkeit von der Sterblichkeitsrate festgelegt. Der Normal-BMI (18,5-24,4 kg/m²) ist der Bereich mit dem geringsten relativen Sterblichkeitsrisiko. Die BMI-Werte 25 und 30 kg/m² sind die entsprechenden risikobezogenen Grenzwerte für Erwachsene für die Feststellung des Übergewichts bzw. der (klinischen) Adipositas.

Gemäß dieser Nomenklatur wird die Adipositas in unterschiedliche Grade aufgeteilt: Adipositas Grad I (BMI= 30-34,9), Adipositas Grad II (BMI=35-39,9), Adipositas Grad III (BMI größer 40) sowie der morbiden Adipositas/Super Adipositas (BMI größer 50).

Übergewicht und Adipositas gehen einher mit Folge- bzw. Begleitkrankheiten (Komorbidität). Sie sind ein wesentlicher Risikofaktor für die Entwicklung eines Typ II Diabetes. Sie bedingen auch häufig soziale Isolation und Diskriminierung. Sie können psychische Störungen unterstützen bzw. auslösen und verringern somit die Lebenserwartung und Lebensqualität. Bei einem BMI größer 30 kg/m² ist die Mortalität (Sterblichkeit) um 1/3, bei 40 kg/m² um den Faktor 3, bei größer 40 kg/m² um den Faktor 3 -20, je nach Gewicht erhöht. Erhöht ist zum Beispiel auch das Risiko einer Krebserkrankung (Gynäkologische Karzinome (Endometrium-Karzinom 4-fach, Mamma- und Zervix-Karzinom 2-fach), Prostata-, Gallenblasen-, Kolon-Karzinom)). Der BMI als Bewertungsmaßstab für eine Adipositas lässt allerdings die Körpergewebszusammensetzung unberücksichtigt. Erhöhte Muskelmasse, Wassergehalt oder Knochenmasse können die Bewertung beeinträchtigen gegebenenfalls berücksichtigt werden. Es kann auch die Körperzusammensetzung aus den Kompartimenten - Fett und fettfreie Masse (Körperwasser wird hier zunächst nicht berücksichtigt) erfasst werden. Nach diesem Zweikompartimentmodell ist der Normalbereich gegeben durch eine Fettmasse < = 20% und eine fettfreie Körpermasse von > = 80%. Ein erhöhter Fettanteil wird als Adipositas beschrieben. Zudem weist eine Erhöhung der Extrazellulärmasse bzw. des Extrazellulärwassers im Verhältnis zur fettfreien Körpermasse auf eine Adipositas hin. Untersuchungen wie die Bioelektrische Impedanz-Analyse (BIA) oder die Infrarot-Spektometrie (NIRI) sind geeignete und in der Praxis angewandte Methoden zur Ermittlung des Körperfettstatus. Ebenso ist die Fettverteilung für die Risikobewertung wichtig. Eine erhöhte abdominale Fettansammlung (zentraler oder androider Verteilungstyp) stellt ein deutlich höheres Risiko dar, als der periphere oder gynoide Verteilungstyp. Daher kann auch die "waist-to-hip"-Ratio (das Verhältnis von Taillenumfang (cm) zu Hüftumfang (cm)) in die Bewertung miteinbezogen werden. Bei Frauen (>0,8) bzw. Männer (>0,95) sind erhöhte Werte mit einer erhöhten Sterblichkeit assoziiert.

Da Adipositas als wesentlicher Risikofaktor für die Entwicklung eines Typ II Diabetes angesehen werden muss, ist die Diagnostik und Therapie der Adipositas bereits im frühen Kindesalter die wichtigste Maßnahme, um zum Beispiel eine Entwicklung des pädiatrischen Typ II Diabetes zu vermeiden. Im Kindes- und Jugendalter kann analog zum Erwachsenenalter der BMI zur Beurteilung von Übergewicht und Adipositas verwendet werden. Da der BMI im Kindes- und Jugendalter entsprechend den physiologischen Änderungen der prozentualen Körperfettmasse von deutlichen alters- und geschlechtsspezifischen Besonderheiten beeinflusst wird, sollte man bei seiner Beurteilung Alter und Geschlecht berücksichtigen. Wegen der geringen Inzidenz von Adipositas-abhängigen Erkrankungen im Kindes- und Jugendalter und mangels ausreichender longitudinaler Untersuchungen zum Gesundheitsrisiko der Adipositas im Kindes- und Jugendalter gibt es im Gegensatz zu der Situation beim Erwachsenen keine festlegbaren Grenzwerte für das gesundheitsgefährdende Ausmaß der Körperfettmasse in diesem Altersbereich. Es kann aber bei der Definition von Übergewicht bzw. Adipositas im Kindes- und Jugendalter das BMI-Perzentil verwendet werden (gewonnen durch Extrapolierung), welches im Alter von 18 Jahren in einen BMI von 25 kg/m² (Übergewicht) bzw. 30 kg/m² (Adipositas) mündet. Es kann zum Beispiel das 90. bzw. des 97. alters- und geschlechtsspezifische Perzentil als Grenzwert zur Definition von Übergewicht bzw. Adipositas verwendet werden. Die BMI-Werte 25 und 30 kg/m² sind die entsprechenden risikobezogenen Grenzwerte für Erwachsene. Bei einem BMI von 25 kg/m² beträgt bei Männern zum Beispiel das relative Risiko für einen Typ II Diabetes 2,2 bei Frauen sogar 5,5.

Eine Behandlung des Übergewichts und der Adipositas hat entweder das Ziel, eine weitere Gewichtszunahme zu verlangsamen, bevorzugt aber zu verhindem, oder ebenso bevorzugt eine Reduktion des Körpergewichts zu erreichen. Dementsprechendes gilt insbesondere für das Ausmaß des Fettgewebsanteils der zu behandelnden Individuen. Umgekehrtes gilt für die Behandlung einer Kachexie oder Lipodystrophie. Eine Behandlung der Übergewichtigkeit bzw. der Neigung zur Adipositas ist, angesichts des bereits in der Übergewichtigkeit erhöhten Risikos für Komorbiditäten, bevorzugt angezeigt für Individuen mit einem BMI größer gleich 25. Insbesondere gilt dies für den Fall, dass Komorbiditäten bereits manifest sind, hier insbesondere bei vorliegenden Anzeichen für die Entwicklung einer Insulinresistenz oder eines Typ II Diabetes. Besonders bevorzugt werden Individuen mit einem BMI größer 30 ohne bzw. mit Komorbiditäten behandelt, die als klinisch adipös bezeichnet werden. Ganz besonders bevorzugt gilt dies für Individuen mit einem höheren Grad der Adipositas.

Der Entwicklung einer Adipositas kann vorgebeugt werden bei Individuen mit normalem BMI oder einem BMI, der diese als übergewichtig qualifiziert, bevorzugterweise dann, wenn es Gründe gibt für die Annahme, dass diese Individuen mit erhöhter Wahrscheinlichkeit zu einer späteren Ausprägung der Adipositas (Erhöhung des BMI über 30 kg/m²), insbesondere assoziiert mit Komorbiditäten, neigen werden.

Solche Gründe können zum Beispiel in einer genetischen Prädisposition, einer nachhaltigen Störung des Lipid- oder Lipoprotein-Stoffwechsels (Dyslipidämien), einem der Adipositas förderlichen und eventuell nur schwer veränderbaren Lebensstil, und/oder einer Adipositas infolge anderer Erkrankungen gegeben sein. Dazu kann eine Diagnostik hilfreich sein, die es erlaubt, beispielsweise genetische Prädispositionen direkt oder indirekt, oder veränderte Gehalte oder Aktivitäten des Rezeptors Fn14 oder dessen natürlichen Liganden oder davon abhängiger Parameter zum Beispiel im Blut oder im Fettgewebe nachzuweisen.

Die im Stand der Technik bekannten Behandlungsverfahren für Adipositas beschränken sich im Wesentlichen auf eine strenge Diät, gekoppelt mit erhöhter Bewegung. Eine bekannte Therapie ist bisher nur über den Noradrenalin-Serotonin-Rezeptoruptake-Inhibitor "Sibutramin" (BASF, Ludwigshafen) oder den Lipasehemmer "Xenical" (Roche, Basel, Schweiz) möglich. Der Einsatzbereich dieser Medikamente ist jedoch auf Grund der auftretenden Nebenwirkungen/Komplikationen eingeschränkt. So ist Sibutramin beispielsweise nicht bei Bluthochdruck - einer typischen Komplikation bei Adipositas - einsetzbar. Zudem kann die Erkrankung nur symptomatisch behandelt werden.

Eine der häufigsten Ko-Morbiditäten der Adipositas ist Diabetes, speziell der Typ II Diabetes.

Beim Diabetes mellitus (allgemein auch als "Zuckerkrankheit" bekannt) handelt es sich um eine chronische Stoffwechselerkrankung, die durch einen erhöhten Blutzuckerspiegel gekennzeichnet ist. Unterschiedliche Ursachen der Erkrankung und auch verschiedene Krankheitsausprägungen erfordern die Unterscheidung von zwei Typen, dem Typ I und dem Typ II Diabetes.

Der Typ I Diabetes (früher: juveniler Diabetes) beginnt meist in der Jugend und entsteht durch eine immunologische Zerstörung der Inselzellen des Pankreas (= Bauchspeicheldrüse). Diese Inselzellen produzieren das Hormon Insulin, das für die Verwertung der Glukose aus der Nahrung verantwortlich ist. Durch die Zerstörung der Inselzellen kommt es zu einem absoluten Insulinmangel. Die Glukose aus der Nahrung kann nicht mehr metabolisiert werden, und der Blutzuckerspiegel steigt. Die Behandlung des Typ I Diabetes geschieht durch die Verabreichung von Insulin.

Der Typ II Diabetes (früher: Erwachsenen- oder Altersdiabetes) entwickelt sich in der Regel im höheren Lebensalter. Er ist dadurch gekennzeichnet, dass die Körperzellen, an denen das Insulin wirken soll, nicht mehr ausreichend auf Insulin reagieren. Dies kann unter anderem auf eine Resistenz von Adipozyten und auch Skelettmuskelzellen gegenüber Insulin zurückgeführt werden. Interessanterweise sind zumeist die älteren, reiferen Adipozyten resistent, während jüngere Adipozyten in der Regel keine Insulin-resistenz aufweisen.

Eine solche Insulinresistenz wird als Folge anhaltend hoher Blutzucker- und Insulinspiegel gesehen, wie sie z.B. bei Übergewichtigen zu beobachten sind. Die Therapie des Typ II Diabetes erfolgt stufenweise: Zunächst wird mit einer Diät versucht, den Blutzuckerspiegel allgemein zu senken. Sind die Diätmaßnahmen zur Behandlung nicht ausreichend, werden in der Folge Blutzucker senkende Medikamente und im fortgeschrittenen Stadium auch Insulin verabreicht.

Die Verabreichung von Insulin kann jedoch bei Patienten mit Typ II Diabetes aufgrund der Insulinresistenz in den Zielgeweben zu Hyperinsulinämie (erhöhter Insulinspiegel in Plasma, Serum) führen. Eine Therapie ist dann mittels Insulin nicht mehr möglich (Curr Diab Rep., Oktober 3 (5), Seite 378-85, (2003)).

Im Rahmen eines von der Anmelderin durchgeführten Projektes zur Identifizierung von Proteinfaktoren mit Relevanz für die Übergewichtigkeit, Adipositas oder Typ II Diabetes oder Kachexie, hat die Anmelderin das Protein TNFSF12 (Synonym: TWEAK) bevorzugt in einer trimeren Anordnung und in seiner löslichen Form als neuen Modulator der humanen Adipozytendifferenzierung entdeckt.

Die Modulation der Adipozytendifferenzierung über eine Aktivierung des TNFSF12 Signaltransduktionssystems wird als therapeutische Strategie unabhängig davon verfolgt, ob das TNFSF12/Fn14 Signaltransduktionssystem in die Disposition, Ätiologie oder in eine Begleiterscheinung der Krankheit involviert ist. Abberante TNFSF12 Expression oder aberrante Fn14 Rezeptoraktivität und/oder Signaltransduktion sind keine Voraussetzungen oder Hindernisse zur Durchführung der hier beschriebenen therapeutischen Verfahren.

Es hat sich ferner überraschenderweise gezeigt, dass dieses Protein TNFSF12 zur Behandlung von Adipositas eingesetzt werden kann. Es konnte gefunden werden, dass TNFSF12 in der Lage ist, die Differenzierung bzw. Funktion von murinen und auch primären humanen Adipozyten reversibel zu hemmen und eine Reduktion von Fettdepots *in vivo* zu erreichen. Dies ermöglicht erstmalig eine Behandlung von Adipositas auf der Ebene der Fettzelldifferenzierung.

Dieses Protein TNFSF12 entfaltet therapeutische Wirkung, bevorzugt durch eine direkte Einwirkung auf bestimmte Fettgewebe innerhalb eines Individuums oder auch indirekt, vermittelt durch andere Fettgewebskompartimente oder auch durch Wechselwirkung über andere Gewebe, die in die Regulation der Energie-Homöostase involviert sind.

Die Erklärung für den gefundenen Phänotyp des Proteins TNFSF12 ergibt sich aus der Eigenschaft von TNFSF12 als Rezeptor-Ligand, welcher bei Bindung Signaltransduktion auslösen kann:

Das Protein TNFSF-12 ist ein Zelloberflächen-assozüertes Typ-II-Membranprotein von 249 Aminosäuren Länge, das ursprünglich als ein Mitglied der Tumor-Nekrose-Faktor (TNF)-Superfamilie beschrieben wurde (Review in: Wiley et al., Cytokine & Growth Factor Reviews, 14, Seite: 241-249, 2003). Ein biologisch aktiver Teil wird als lösliches Protein mit einer Länge von etwa 156 Aminosäuren in das extrazelluläre Millieu abgespalten, und kann seinerseits den korrespondierenden Rezeptor aktivieren. Diese Rezeptoraktivierung selbst kann in der Zelle Veränderungen auslösen, beispielsweise Zellteilung fördern, zelluläre Migration fördern und zur Sekretion von Zytokinen führen, die im Zusammenhang mit entzündlichen oder zytotoxischen Prozessen diskutiert werden.

Ursprünglich wurde als Rezeptor für TNFSF12 das Zelloberflächenmolekül DR3 beschrieben (Marsters et al., Curr Biol, 8, Seite: 525-528, 1998). Später wurde dann nachgewiesen, dass DR3 nicht der physiologische Rezeptor für TNFSF12 ist (Schneider et al., Europ J Immun, 29, Seite: 1785-1792, 1999, und Kaptein et al., FEBS Lett, 485, Seite:135 - 141, 2000). Anstelle dessen ist nach dem derzeitigen Stand der Technik nur ein Rezeptor für TNFSF-12 bekannt, der von TNFSF12 mit physiologischer Aktivität gebunden wird (Wiley et al., Immunity, 15(5), Seite:837 - 846, 2001). Dieser Rezeptor, ein Mitglied der TNF-Rezeptor-Superfamilie, wird als "TWEAK-R" oder "fibroblast growth factor inducible 14" (Fn14) beschrieben (Meighan-Mantha et al., J Biol Chem, 274, Seite:33166 - 33176, 1999; Wiley et al., Cytokine & Growth Factor Reviews, 14, Seite:241 - 249, 2003). Dieser Rezeptor ist das kleinste Mitglied aus der Gruppe der TNF Rezeptoren. Derzeit sind keine andere Liganden bekannt, die an Fn14 binden (Wiley et al., Cytokine & Growth Factor Reviews, 14, Seite:241 - 249, 2003). Allerdings werden auch noch andere mögliche Rezeptoren für TNFSF12 diskutiert (Polek et al., J Biol Chem., 278, Seite 32317-23, 2003). Es wurde aber auch beschrieben, dass TNFSF12 nicht an andere bekannte TNF-Rezeptoren bindet (Wiley et al., Cytokine & Growth Factor Reviews, 14, Seite:241 - 249, 2003).

Die Primärsequenz von Fn14 umfasst 129 Aminosäuren, wobei der Aminoterminus des Rezeptors prozessiert wird, so dass das reife Protein nur noch aus 102 Aminosäuren besteht.

Für TNFSF-12 sind bisher in der Literatur verschiedene zelluläre Aktivitäten beschrieben worden, so z.B. die Induktion von Proliferation und Migration von Endothelzellen, die in der Angiogenese eine zentrale Rolle spielen.

Auch die Induktion der Expression von inflammatorischen Zytokinen, wie beispielsweise IL-6, IL-8 und RANTES ist unter anderem in Fibroblasten beschrieben worden (Chicheportiche, Arthritis Res, 4, Seite:126 - 133, 2002).

Unter besonderen Bedingungen (z.B. Kostimulation mit IFN-gamma) wurde die Stimulation von Zelltod in einigen sensitiven Tumorzellen beschrieben (Chicheportiche et al., J Biol Chem. 272, Seite:32401 - 32410, 1997; Nakayama et al., J Immunol, 170(1), Seite: 341 - 348, 2003). Die zyototoxische Aktivität von Monozyten *in vitro* hängt teilweise von ihrer TWEAK-Expression und dessen parakriner Wirkung auf solche Tumorzellen ab (Nakayama et al., J Exp Med, 192, Seite:1373 - 1380, 2000).

Diese oben genannten Beobachtungen führten dazu, TNFSF12 im Zusammenhang mit angiogenetischen und auch inflammatorischen Krankheitsprozessen zu diskutieren.

Unter experimentellen Bedingungen, das ist die Öffnung der Blut-Hirn-Schranke durch geeignete Methoden, konnte *in vivo* eine Beteiligung von transgenem löslichem TNFSF12 an inflammatorischen Prozessen des zentralen Nervensystems dargestellt werden. Dies beruht vermutlich auf der Stimulation der Proliferation von Astrozyten (Desplat-Jego et al., J Neuroimmunol, 133, Seite:116 - 123, 2002). Natürlicherweise gelangt TWEAK dann über die Blut-Hirn-Schranke, wenn es dort von infiltrierenden Leukozyten exprimiert wird.

Demgegenüber wurde eine negative Regulation von TWEAK-mRNA *in vivo* in Lipopolysaccharid-induzierter inflammatorischer akuter und chronischer Reaktion und Tiermodellen für Autoimmunkrankheiten beobachtet (Chicheportiche et al., Biochem Biophys Res Comm, 279, Seite:162-165, 2000).

TWEAK-vermittelte Aktivitäten als mögliche Ursache für Krankheitsprozesse wurden auch in der Patentanmeldung WO03/086311 auf der Grundlage von Daten aus Zellkulturen und transgenen Mäusen beschrieben.
Die transgenen Tiere exprimierten dabei entweder ein zunächst zellgebundenes Volle-Länge TWEAK Protein oder ein lösliches TWEAK-Proteinfragment.
Die transgene Expression von Volle-Länge Tweak-Protein führte zu Thrombosen in den Herzkammem und zu deren Dilatation, zu Hyperplasien von Zellen des Gallenganges und ovaler Zellen in der Leber sowie zu verstärkter Vakuolisierung und Zelltod von Leberzellen. In den Nieren kam es zu Zystenbildungen in den Nierenglomeruli. In der Lunge wurden Granulomatosen und die Penetration von Lymphozyten im Rahmen einer Inflammation erkannt.
Lösliches TWEAK führte bei hohen Dosen sogar zum Tod der Tiere einige Monate nach der Geburt. Selbst niedrige Dosen führten zum Beispiel zu Veränderungen der zellulären Struktur des Herzens (dilatative Kardiomyopathie) sowie eine tubuläre Hyperplasie und Glomerulopathie in den Nieren.

Konsequenterweise wurden in WO03/086311 antagonistische therapeutische Strategien verfolgt, die eine Inhibition der TWEAK abhängigen Signalgebung zum Ziel haben. Als Beispiele hierfür wurde bei Nieren mit Zeichen für ein Alport Syndrom, eine genetische inflammatorische Erkrankung, die unter anderem durch Ausdünnung und schließlich Zerstörung der Glomerulimembranen durch fibrotisches Ersatzgewebe gekennzeichnet ist, angegeben. Es zeigte sich weiter nach WO03/086311 eine erhöhte Fn14 Expression, und eine Behandlung von Tieren mit derartigen Nierenveränderungen mit TWEAK-Antagonisten (antagonistische Antikörper) führt zu einer Verringerung der krankhaften fibrotischen Veränderungen.

Nach dem Stand der Technik, insbesondere nach WO03/086311, scheint eine erhöhte Expression von TWEAK Protein *in vivo* mit pathologischen Befunden im Herz, der Leber, der Niere und der Lunge zu korrelieren, was in der therapeutischen Anwendung eine Blockierung der TWEAK abhängigen Aktivitäten verlangt.

In WO03/086311, wurde ebenfalls beschrieben, dass *in vitro* die Exposition von murinen Prä-Adipozyten (3T3-L1 Zellen) zu TNFSF12 mit deren Fähigkeit, intrazellulär Lipid einzulagern, negativ interferieren kann. Der Mechanismus ist unklar, da *in vivo* nicht gezeigt wurde, ob FN14 der einzige Rezeptor für Fn14 ist und ob eine Expression von Fn14 in 3T3-L1 Zellen oder auch in Fettgewebe vorlag. Dies ist insbesondere deshalb relevant, da von Polek et al. (J Biol Chem., 278, Seite 32317-23, 2003) weiterhin die Möglichkeit mehrerer Rezeptoren für TWEAK diskutiert wird.

Humane Adipozytendifferenzierung ist bekanntermassen nicht ohne weiteres durch murine Zellkultursysteme darstellbar. So zeigen beispielsweise murine Präadipozyten typischerweise eine sogenannte klonale Expansion. Dieses ist eine Zellzahlvermehrung durch kurzzeitigen Wiedereintritt in den Zellzyklus nach Induktion zur Differenzierung in zuvor bereits postmitotischen Zellen. Dieses Phänomen zeigen differenzierende humane Adipozyten nicht. Faktoren, die eine klonale Expansion inhibieren, können in murinen Adipozyten die Differenzierung blockieren. Es ist deshalb nicht klar, ob differenzierungsinhibierende Faktoren, die anhand muriner Adipozytenzellkulturen identifiziert worden sind, auch in primären humanen Adipozyten eine Blockade der Differenzierung erreichen können.
Darüberhinaus führen beispielsweise Expositionen mit dem Protein TNF-alpha in sich differenzierenden murinen Adipozytenzellkulturen dosisabhängig zu Zelltod, während primäre humane Adipozyten nach ebensolcher Exposition keinen solchen Zelltod aufweisen, wohl aber in ihrer Differenzierung inhibiert sind.
An murinen Zellkulturen gewonnene insbesondere funktionelle Daten lassen sich demnach nicht auf humane Zellkulturen übertragen.
Darüberhinaus ist für eine therapeutische Anwendbarkeit hauptsächlich der Nachweis des gewünschten therapeutischen Effekts, hier der Gewichtsreduktion, *in vivo* relevant. Denn es ist bekannt, dass viele Phänomene, die in Zellkulturen zu beobachten sind, in komplexeren lebendigen Organismen nicht stattfinden oder in ihrer Bedeutung irrelevant sind.

Im Rahmen der vorliegenden Erfindung konnte neben den bekannten oben beschriebenen Wirkungen des TNFSF12/Fn14 Liganden-Rezeptorsystems eine dosisabhängige und reversible Inhibition der Adipozyten-Differenzierung bzw. Hemmung der Lipideinlagerung in humanen Adipozyten als Resultat der durch TNFSF12 verursachten Fn14-Signaltransduktion gezeigt werden. Diese Inhibierung der Differenzierung von Adipozyten konnte von der Anmelderin überraschenderweise in frischen menschlichen Fett-Zellen (bzw. Fett-Vorläuferzellen) gezeigt werden.
Ferner war es durch die Arbeiten der Anmelderin erstmals möglich zu zeigen, dass die Aktivierung des TNFSF12/Fn14 Liganden-Rezeptorsystems sowohl in Zellkultur als auch durch rekombinante Zurverfügungstellung von TNFSF12 in murinen Tiermodellen *in vivo* mit einer Reduzierung des Gewichtes und Fettgehalts von Tieren einhergeht.

Diese *in vivo* Effekte auf Tiergewicht und Fett wurden ohne assoziierte massive entzündliche Prozesse oder Toxizitäten in diesen Tierexperimenten beobachtet. Dieses war sehr überraschend, da das Auftreten von entzündlichen Prozessen oder Toxizitäten nach der WO03/086311 zu erwarten war.
Ferner konnte von der Anmelderin erstmals gezeigt werden, dass agonistische Antikörper, welche den Rezeptor Fn14 binden und aktivieren, einen direkten Effekt auf die Fettzelldifferenzierung auslösen und die Fettzelldifferenzierung inhibieren.

Aufgrund des von der Anmelderin gezeigten Effekts des TNFSF12/Fn14 Liganden-Rezeptorsystems auf die Differenzierung von Fettzellen und das Gewicht und den Fettgehalt von Tieren, ist es vorteilhaft, diese Effekte durch die Fn14-abhängige Signaltransduktion induzierten Phänotypen für therapeutische Anwendungen zu nutzen. Dadurch ist eine therapeutische Verwendung der Fn14-Aktivierung zur Vorbeugung und/oder Behandlung von Adipositas und/oder Adipositas assoziierten Krankheiten möglich.

In Analogie zu TNF-R und anderen TNF-ähnlichen Rezeptoren wird angenommen, dass die Aktivierung des Fn14-Signalweg vorzugsweise dann auftritt, wenn zahlreiche Rezeptoren auf der Zelloberfläche in nächster Nähe exprimiert werden. Dieser Prozess ist bekannt als Rezeptorklusterbildung. Die TNF-Liganden sind multivalente Komplexe, die gleichzeitig an mehr als einen Rezeptor binden und auf diese Weise aggregieren können. Rezeptorklusterbildung als Mittel zur Rezeptoraktivierung in anderen Systemen ist ausführlich beschrieben worden, insbesondere für die Tyrosinkinase (Ullrich und Schlessinger, Cell, 61, Seite 203 - 212 (1990); Kolanus et al., Cell, 74, Seite 171 - 183 (1993)). Demnach könnte die Gabe von Fn14-Liganden und / oder Fn14-aktivierenden Agenzien, welche beispielsweise auch Antikörper, Antikörperderivate, Bindeproteine oder Bindemoleküle umfassen, die die Klusterbildung und/oder eine stromabwärts gelegene Signalgebung auf der Oberfläche von Zielzellen induzieren können, für eine direkte Stimulierung des Fn-14-Signalwegs in diesen Zellen nutzbar sein.

Der Signalweg durch FN-14 ist bekannt dafür, dass er unter anderem Reaktionswege aktiviert, die potentiell immunologische oder entzündliche oder zytotoxische Vorgänge modulieren können. Diese Eigenschaften der Fn14-Aktivierung, sowie die Möglichkeit, diese Effekte durch antagonistische Antikörper zu hemmen, stellt auch die Basis für die in der Patentanmeldung von Biogen WO03/086311 beschriebene Verwendung von Fn14-blockierenden Agenzien, einschliesslich spezifischer antagonistischer Fn14-Antikörper, für die Behandlung von Krankheiten dar, welche mit immunologischen oder entzündlichen oder zytotoxischen Vorgängen assoziiert sind. Ebenfalls beschrieben ist in WO03/086311 die Verwendung von agonistischen Antikörpern, welche eine Induktion der Fn-14 Signaltransduktion bewirken. Ob sich eine solche Aktivierung allerdings durch induzierte immunologische oder entzündliche oder zytotoxische Vorgänge schädlich auf normale Gewebe auswirkt, ist im Stand der Technik nach wie vor ungeklärt.

Liganden, wie TNFSF12, welche den Rezeptor Fn14 aktivieren, können die Fettzelldifferenzierung direkt über Liganden/Rezeptorwechselwirkungen und dadurch ausgelöste Rezeptoraktivierung und Signaltransduktion hemmen. Da Rezeptoraktivierung auch durch agonistische Antikörper erzielt werden kann, beispielsweise durch solche, die den entsprechenden Rezeptor Fn14 erkennen und bei Erkennung die Signaltransduktion induzieren, können anstelle des natürlichen Liganden auch agonistische Antikörper verwendet werden, welche den oben beschrieben Rezeptor Fn14 gezielt und spezifisch aktivieren.

Der durch die Experimente der Anmelderin gefundene Zusammenhang der Funktionalität des Fn14-Signalweges mit der Differenzierung von Adipozyten kann zur Generierung von neuen Therapeutika zur Behandlung von Adipositas und Adipositas-assoziierten Krankheiten genutzt werden.

Wie bereits oben beschrieben wurde, stellt die Behandlung von Adipositas und der mit Adipositas assoziierten Krankheiten ein enormes klinisches Problem dar. Bisherige Therapieansätze erfolgten und erfolgen derzeit nur symptomatisch entweder über veränderte Emährung und, im Falle von Adipositas-assozüerter Diabetes, beispielsweise über die Verabreichung von PPAR-gamma Agonisten. Als Liganden für PPAR-gamma aktivieren diese den PPAR-gamma Transkriptionsfaktor, der eine Differenzierung der Adipozyten oder eine Aktivierung des Fettstoffwechsels in reifen Adipozyten bewirken kann. Nachteile solcher symptomatischen Behandlungen (u.a. der Diabetes) sind zudem das Auftreten von Folgeerscheinungen, wie beispielsweise kardiovaskulären Komplikationen, diabetischen Nephropathien, Neuropathien oder Retinopathien, die zusätzlich therapiert werden müssen. Alle bisher bekannten therapeutischen Ansätze haben zudem den folgeschweren Nachteil, dass diese nicht in der Lage sind, die Differenzierung bzw. Funktion von Adipozyten zu hemmen.

Eine Aufgabe der vorliegenden Anmeldung ist es daher Substanzen zur Behandlung und / oder Vorbeugung von Adipositas, und damit auch zur Vorbeugung von Adipositas-assoziierter Diabetes (Typ II) bereitzustellen. Diese Substanzen umfassen insbesondere agonistische anti-Fn14 Antikörper, Antikörperderivate, Bindeproteine oder Bindemoleküle. Da aus dem bisher vorhandenen Stand der Technik abgeleitet werden kann, dass die Aktivierung von Fn14-Signaltransduktion entzündliche und toxische Effekte in Organismen verursachen können, ist eine spezielle Ausführung der vorliegenden Anmeldung Substanzen (insbesondere agonistische anti-Fn14-Antikörper, Antikörperderivate, Bindeproteine oder Bindemoleküle) zur Behandlung und / oder Vorbeugung von Adipositas, und damit auch zur Vorbeugung von Adipositas-assoziierter Diabetes (Typ II) bereitzustellen, welche in wirksamen Dosen nicht mit relevanten der Therapie entgegenstehenden Toxizitäten verbunden sind.

Allerdings ist die zielgerichtete Herstellung von solchen agonistischen anti-Fn14-Antikörpern, Antikörperderivaten, Bindeproteinen oder Bindemolekülen problematisch, welche als gemeinsame Eigenschaft die Fähigkeit haben, durch Bindung an Fn14 die Signaltransduktion zu initiieren. So ist es bekannt, dass in vielen Fällen Bindung von Antikörpern, Antikörperderivaten, Bindeproteinen oder Bindemolekülen allein nicht ausreichend ist, um den Rezeptor zu aktivieren. (Mueller et al., 2005, J Neuroimmunol., 159:55-65). Vielmehr muss die Bindung solcher Moleküle das molekulare Ereignis, welches bei Bindung des natürlichen Liganden an den Rezeptor stattfindet, ebenfalls auslösen. Solche Ereignisse können beispielsweise die Auslösung von Änderungen in Rezeptorstrukturen und/oder Multimerisierungen von Rezeptoren sein. Hierbei ist es häufig zu beobachten, dass nur die spezifische Bindung an wenige definierte Stellen des Rezeptors eine optimale Rezeptoraktivierung bewirken kann. Wenn allerdings diese Stellen des Rezeptors, welche bei Bindung zur Aktivierung führen können, nicht bekannt sind, gestaltet sich die Generierung von aktivierenden Agenzien sehr schwierig, insbesondere dann, wenn kein aussagekräftiges biologischen Testsystem zur Verfügung steht.

Eine weitere Aufgabe der vorliegenden Anmeldung ist es daher, die für eine Aktivierung notwendigen Bindestellen bzw. Epitope auf dem Rezeptor herauszufinden, und basierend auf diesem bisher nicht verfügbarem Wissen Methoden und Verfahren zu entwickeln, welche eine effiziente und gezielte Gewinnung von aktivierenden Antikörpern ermöglichen. Basierend auf dem generierten Wissen über die für eine Aktivierung notwendigen Bindestellen bzw. Epitope auf dem Rezeptor ist es dann eine weitere Aufgabe der vorliegenden Anmeldung, Substanzen, insbesondere, agonistische anti-Fn14-Antikörper, Antikörperderivate, Bindeproteine oder Bindemoleküle zu identifizieren, welche die für eine Aktivierung notwendigen Bindestellen bzw Epitope auf dem Rezeptor binden und so die Rezeptor-Signaltransduktion aktivieren, und diese als Mittel zur Behandlung und / oder Vorbeugung von Adipositas, und damit auch zur Vorbeugung von Adipositas-assozüerter Diabetes (Typ II) bereitzustellen.

Erfindungsgemäß wird das beschriebene Problem der Behandlung und / oder Vorbeugung von Adipositas gelöst durch die Generierung von Substanzen, welche die Differenzierung bzw. Funktion von Adipozyten nachhaltig hemmen. Diese Substanzen zeichnen sich erfindungsgemäss durch ihre Fähigkeit aus, Fn14-vermittelte Signaltransduktion zu induzieren. Beispiele für Agenzien, welche Fn14-vermittelte Signaltransduktion induzieren, sind die natürlichen Liganden oder homologe Liganden (oder spezies-fremde Liganden) von Fn14, sowie Antikörper, welche Fn14 an definierten Positionen (Epitopen) erkennen und durch Bindung an mindestens eines dieser Epitope aktivieren. Die Verwendung von Antikörpern, welche Fn14 erkennen und aktivieren, hat insofern Vorteile, als dass diese sowohl einfacher herzustellen sind, als auch aufgrund deren guten pharmakologischen Verhaltens kontrollierter im Patienten anzuwenden sind.

Die Erfindung umfasst ebenfalls die Epitope auf dem Rezeptor Fn14, welche bei Bindung das molekulare Ereignis, welches bei Bindung des natürlichen Liganden an den Rezeptor stattfindet, ebenfalls auslösen, und damit zur Rezeptoraktivierung und nachfolgender Modulation der Adipozytendifferenzierung führen.

Das beschriebene Problem der Bereitstellung von Agenzien zur Behandlung und / oder Vorbeugung von Adipositas wird gelöst durch die erfindungsgemäßen Agonisten, wie Antikörper bzw. Derivate davon, die den Rezeptor Fn14 erkennen und aktivieren. Diese agonistischen Antikörper und Zusammensetzungen werden gezielt hergestellt durch Techniken und Verfahren, welche auf dem Wissen über die für Rezeptoraktivierung notwendigen Positionen (Epitope) auf Fn14 basieren. Diese agonistischen Antikörper und Zusammensetzungen, diese enthaltend, werden eingesetzt zur Behandlung von Adipositas und weiteren metabolischen Krankheiten, die mit gesteigerter oder aberranter Menge an Fettzellen assoziiert sind, indem der Fn14-Signalweg stimuliert wird.

Eine bevorzugte Ausführungsform der Erfindung beschreibt die Verwendung von wenigstens einem Antikörper, der gegen Fn14 (anti-Fn14 AK) gerichtet ist, zur Modulierung der Fettzelldifferenzierung, bevorzugt ist die Verwendung eines monoklonalen oder rekombinanten Antikörpers (anti-Fn14 mAK bzw. anti-Fn14 rekAK). Eine weitere Ausführungsform der Erfindung beschreibt Antikörper oder Antikörperderivate, die gegen spezifische Epitope des Rezeptors, welche bei Bindung durch Antikörper die Aktivierung des Rezeptors verursachen, gerichtet sind.

Eine weitere Ausführungsform der Erfindung beschreibt Bindemoleküle (beispielsweise Affiline, Antikaline, Aptamere), die gegen solche spezifische Epitope des Rezeptors, welche bei Bindung durch Antikörper die Aktivierung des Rezeptors verursachen, gerichtet sind.

Eine weitere Ausführungsform der Erfindung beschreibt spezifische Epitope des Rezeptors, welche bei Bindung durch Antikörper, Antikörper-Derivate, Bindeproteine oder Bindemoleküle die Aktivierung des Rezeptors verursachen.

Die Erfindung stellt weiterhin ein neues Screening-Verfahren zur Selektion von Fn14-aktivierenden Agenzien dar, wobei diese Agenzien u.a. Anti-Fn14-Antikörper, - Derivate, Bindeproteine und Bindemoleküle umfassen, und durch Bindung an mindestens ein aktivierendes Epitop des Fn14 die Aktivierung des Fn14 ermöglichen.

Der dabei verwendete Ansatz benutzt beispielsweise Antikörper gegen Epitope des Rezeptors, welche bei Bindung durch Antikörper die Aktivierung des Rezeptors verursachen und den Rezeptorsignalweg auslösen. Dieses kann entweder direkt durch Messung von Signaltransduktionsereignissen oder indirekt, z.B. durch Analyse der Modulation zellulärer Phänotypen, durch beispielsweise Inhibierung der Fettzelldifferenzierung gemessen werden.

Das verwendete Verfahren, um beispielsweise die agonistischen Eigenschaften und Anwendbarkeiten in der Modulation der Fettzelldifferenzierung von möglichen agonistischen Antikörper für Fn14 zu testen, wird anhand von Beispielen beschrieben und umfasst vorzugsweise die folgenden Schritte:
1) Undifferenzierte humane Präadipozyten werden subkonfluent in Zellkulturschalen ausgesät und bis zur Postkonfluenz kultiviert.
2) Diese Zellen werden mit spezifischen Differenzierungsinduktoren inkubiert, was im Normalfall zur messbaren Differenzierung in Adipozyten mit eingelagertem Fett führt.
3) Simultan werden die derart zur Differenzierung angeregten Zellen mit zu testenden Agenzien, wie Anti-Fn14-Antikörpern, -Antikörperpräparationen oder - Formulierungen inkubiert.
4) Die Messung der Differenzierung der Zellen erfolgt beispielsweise nach >= 8 Tagen mit einem Test, der zur Detektion von reifen Fettzellen geeignet ist (beispielsweise mit einem Lipidnachweistest, z.B. Nile Red Assay, wie in Beispiel 1 beschrieben).
5) Agenzien, welche die Fettzelldifferenzierung modulieren, werden dadurch erkannt, dass in diesen Zellen eine Abweichung der Test-Signalstärke stattfindet, welche mehr als eine einfache Standardabweichung beträgt, vorzugsweise eine mehr als 2-fache Änderung vom Signal, welches mit negativen Kontrollantikörpem erhalten wird.
6) Die Stärke der differenzierungsmodulierenden Aktivitäten der getesteten Agenzien, die im vorhergehenden Schritt positiv waren, wird durch serielle Verdünnungen und darauffolgende Bestimmungen dosisabhängiger Phänotypänderungen analysiert. Die Spezifitäten der Antikörper für aktivierende Epitope werden daraufhin durch direkte Bindungseperimente, beispielsweise ELISA mit immobilisierten Peptiden, welche aktivierende Epitope darstellen, bestätigt.

Dieses Verfahren, welches nur beispielhaft gelistet wurde, kann an das Ziel, weitere neue Fn14-aktivierende Antikörper, welche Epitope erkennen, die bei Bindung die Aktivierung des Rezeptors verursachen, zu identifizieren, auf verschiedene Weisen moduliert und angepasst werden.

So können beispielsweise anstelle differenzierender Präadipozyten für das beschriebene Verfahren zur Generierung von differenzierungsmodulierenden Agenzien auch andere Zellen oder Zelllinien, welche Fn14, natürlicherweise, in rekombinanter Form als Chimären-Rezeptor, oder entsprechende Epitop-haltige Moleküle exprimieren, verwendet werden. Bei der Verwendung solcher Zellen anstelle der Präadipozyten, kann der Fn14 aktivierende Effekt von Agenzien beispielsweise über den Nachweis von spezifischen Signaltransduktionsereignissen in der Zelle festgestellt werden.

Ein anti-Fn14-Antikörper (oder eine Kombination von Antikörpern), der die Differenzierung von Adipozyten inhibiert oder moduliert, ist in dieser Versuchsanordnung ein Fn14-aktivierendes Agenz. Diese Agenzien werden zur Herstellung einer pharmazeutischen Zusammensetzung zur Behandlung von Adipositas und weiteren metabolischen Krankheiten, die mit gesteigerter oder aberranter Menge an Fettzellen assoziiert sind, verwendet.

Wie oben ausgeführt betrifft die Erfindung ebenfalls die Verwendung von funktionellen Varianten der oben aufgeführten Antikörper zur Herstellung einer pharmazeutischen Zusammensetzung zur Behandlung von Adipositas und weiteren metabolischen Krankheiten, die mit gesteigerter oder aberranter Menge an Fettzellen assoziiert sind.

Die Erfindung betrifft ebenfalls die Verwendung von Substanzen und Präparationen, welche aktivierenden Epitope von Fn14 darstellen, zur direkten Immunisierung von Patienten, zur Vorbeugung und/oder Behandlung von Adipositas und/oder weiteren Adipositas-assoziierten Krankheiten, die mit gesteigerter oder aberranter Menge an Fettzellen assoziiert sind.

### Detaillierte Beschreibung der Erfindung

Die nachfolgende Beschreibung soll bevorzugte Ausführungsformen der Erfindung beispielhaft darlegen.

### Definitionen

*Der Begriff* "Adipozyten-Differenzierung" bezieht sich auf die Fähigkeit einer entsprechend determinierten Vorläuferzelle (Präadipozyt), sich auf geeignete Stimuli hin terminal zu differenzieren. Dabei wird ein spezifisches Genexpressionsprogramm initiiert, das phänotypisch unter anderem zu einer intrazellulären Akkumulation von Lipid führt. Eine derart differenzierter Adipozyt wird auch als reifer Adipozyt bezeichnet.

*Der Begriff* "Metabolisches Syndrom" bezieht sich auf die variable Kombination von klinischen Parametern und Symptomen, die u.a. mit Adipositas assoziiert sind. Dieses schliesst unter anderem häufig Adipositas und/oder Bluthochdruck und/oder hohe Cholesterinwerte und/oder Insulinresistenz ein.

Der Begriff "Epitop" (oder Antikörper-Bindestelle) ist definiert als die räumliche Struktur eines Antigens, die von einem Antikörper erkannt und gebunden wird.

Ein monoklonaler Antikörper (mAK) erkennt ein einziges Epitop, da alle Antikörper von einer produzierenden B-Zelle (= eine antikörperproduzierende Zelle) abstammen und demnach diesselbe Struktur aufweisen. Polyklonale Antikörper (pAK) sind Gemische verschiedener Antikörper, synthetisiert durch verschiedene B-Zellklone, welche verschiedene Epitope erkennen können, da sie unterschiedliche Antigenbindungsstellen aufweisen. Ein Epitop umfasst in der Regel mehrere Aminosäuren (beispielsweise 5-7 Aminosäuren), die sich in linearer Abfolge in der Primärzequenz des Antigens befinden können (= kontinuierliche Epitope oder lineare Epitope), oder aus verschiedenen, nicht unmittelbar verknüpften Aminosäuren des Antigens bestehen können (= diskontinuierliche Epitope, nicht-lineare Epitope).

*Der Begriff* "Fc-Domäne" eines Antikörpers bezieht sich auf einen Teil eines Moleküls, der die CH2-, CH3- und Hingeregionen umfasst, dem jedoch die Antigenbindestellen fehlen.

*Der Begriff* "Fn14-aktivierendes Agenz" bezieht sich auf jedes Agenz, das in der Lage ist, Ligandenbindung an Fn14 zu imitieren, Klusterbildung von Fn14 auf der Zelloberfläche oder den Fn14-Signalweg zu ermöglichen oder verstärken oder das Einfluß darauf ausüben kann, wie innerhalb der Zelle das Fn14-Signal interpretiert wird. Beispiele für Fn14-aktivierende Agenzien sind TNFSF12, lösliche anti-Fn14-Antikörper, kreuzverknüpfte anti-Fn14-Antikörper sowie multivalente anti-Fn14-Antikörper.

*Der Begriff* "Fn14-Signalweg" bezieht sich auf alle molekularen Reaktionen, die im Zusammenhang mit der Liganden-, Antikörper- oder Bindepartner-vermittelten Aktivierung des Fn14 und den daraus resultierenden molekularen Reaktionen stehen.

Der *Begriff* "anti-Fn14-Antikörper ("anti-Fn14-AK") bezieht sich auf alle Antikörper, die wenigstens ein Epitop des Fn14 erkennen und daran binden.

*Der Begriff "anti-Fn14-monoklonaler* Antikörper" ("anti-Fn14mAK") bezieht sich auf alle monoklonalen Antikörper, die ein einziges Epitop des Fn14 erkennen und daran binden.

*Der Begriff* "anti-Fn14-Antikörper, poly- oder monoklonal, kreuzverknüpfendes Agenz" bezieht sich auf alle Agenzien, die entweder kovalent oder nicht-kovalent anti-Fn14-Antikörper in Lösung binden können, so dass die Antikörper an der Oberfläche potentieller Zielzellen binden und dort Rezeptor-Signaltransduktion induzieren. Dieses schliesst auch Antikörper ein, welche die Rezeptoren aggregieren können, so dass die Antikörper an der Oberfläche potentieller Zielzellen binden und dort Rezeptorklusterbildung vervielfachen können.

*Der Begriff* "funktionelle Variante eines Antikörpers" bezieht sich erfindungsgemäss auf einen Antikörper und/oder Fragment, das im wesentlichen die biologische Funktion oder Funktionen des Antikörpers vermittelt. Im Falle der vorliegenden Antikörper kann es sich hierbei um die Fähigkeit handeln, die Lipideinlagerung in geeigneten Zellen zu inhibieren. Unter diesen Begriff fallen ebenfalls verschiedene Derivate von Antikörpern, insbesondere rekombinante, chimäre, humanisierte, oder anderweitig modifizierte Antikörper, welche die Fn14-Signaltransduktion induzieren.

*Der Begriff* "funktionelle Variante" schliesst auch "Nicht-Antikörper"-Proteine mit ähnlichen Bindeeigenschaften ein, beispielsweise Antikaline, Affiline, EinzeldomänenAntikörper und andere spezifische Bindeproteine.

*Der Begriff* "funktionelle Variante" eines Polypeptids oder einer Nukleinsäure bezieht sich vorzugsweise auf Polypeptide oder Nukleinsäuren mit einer Sequenzähnlichkeit, insbesondere einer Sequenzidentität von etwa mindestens 25%, bevorzugt etwa 40%, insbesondere etwa 60%, besonders bevorzugt etwa 70%, ganz besonders bevorzugt etwa 80%, insbesondere etwa 90% und am meisten bevorzugt von 98% mit dem Polypeptid. Solche Varianten sind beispielsweise homologe Polypeptide, die aus anderen Organismen stammen. Andere Beispiele von Varianten sind Polypeptide oder Fragmente, die durch unterschiedliche Allele eines Gens kodiert werden. Funktionelle Varianten schließen vorzugsweise auch natürlich vorkommende Mutationen ein, insbesondere Mutationen, welche in quantitativer Weise die Aktivität der Peptide verändern, die durch diese Sequenzen kodiert werden. Ferner können solche Varianten vorzugsweise aus einem differenziellen Splicen der kodierenden Gene resultieren. In einer besonders bevorzugten Ausführungsform schließt der Ausdruck "funktionelle Variante" Derivate mit Einzelnukleotidpolymorphismen ("single nucleotide polymorphism" (SNP)) ein.

*Der Begriff* "Sequenzidentität" bezieht sich auf den Identitätsgrad (% Identität) zweier Sequenzen, die im Fall von Polypeptiden beispielsweise durch BLASTP 2.2.10 und im Fall von Nukleinsäuren beispielsweise durch BLASTN 2.2.10 bestimmt werden kann, wobei der niedrige Komplexitätsfilter (low complexity filter) ausgeschaltet ist und im Falle von BLASTP die Matrix BLOSUM 62 ist (Altschul et al., 1997, Nucleic Acids Res., 25:3389-3402).

*Der Begriff* "Sequenzähnlichkeit" oder "Sequenzhomologie" bezieht sich auf die Ähnlichkeit (% Positive) zweier Nukleotid- bzw. Polypeptidsequenzen, bestimmt beispielsweise mittels BLASTN 2.2.10 bzw. BLASTP 2.2.10, wobei der Filter ausgeschaltet ist und im Falle von BLASTP die Matrix BLOSUM 62 ist (Altschul et al., 1997, Nucleic Acids Res., 25:3389-3402).

*Der Begriff* "Bindungsprotein" oder "Bindungspeptid" bezieht sich auf eine Klasse von Proteinen, Peptiden oder Fragmenten, welche das jeweilige Molekül binden oder inhibieren, einschließlich, ohne Beschränkung, polyklonale oder monoklonale Antikörper, Antikörperfragmente und Proteingerüste, die gegen diese Proteine, Peptide oder Fragmente gerichtet sind.

*Der Begriff* "Aptamer" beschreibt Nukleinsäuren, die mit hoher Affinität an ein Polypeptid binden. Aptamere können durch Selektionsverfahren wie SELEX (siehe z.B. Jayasena, Clin. Chem., 45, Seite 1628 - 1650, (1999); Klug und Famulok, M. Mol. Biol. Rep., 20, Seite 97-107, (1994); US 5,582,981) von einem großen Pool unterschiedlicher Einzelstrang-RNA-Moleküle isoliert werden. Aptamere können auch in ihrer Spiegelform synthetisiert und selektiert werden, beispielsweise als das L-Ribonukleotid (Nolte et al., Nat. Biotechnol., 14, Seite 1116 - 1119, (1996); Klussmann et al., Nat. Biotechnol., 14, Seite 1112 - 1115, (1996)). Formen, die auf diese Weise isoliert wurden, haben den Vorteil, dass sie nicht durch natürlich vorkommende Ribonukleasen abgebaut werden und daher eine größere Stabilität aufweisen.

### Herstellung von anti-Fn14-Antikörpern zur Modulation der Differenzierung von Adipozyten

Das generelle Verfahren zum Herstellen eines Antikörpers oder Antikörperfragments wird mittels Verfahren durchgeführt, die dem Fachmann bekannt sind, beispielsweisedurch Immunisieren eines Säugers, beispielsweise eines Kaninchens, mit dem entsprechenden Antigen, wobei, falls erforderlich, entsprechende Adjuvantien, beispielsweise Freunds Adjuvans und/oder Aluminiumhydroxidgele, oder andere Adjuvanzien verwendet werden können (siehe beispielsweise Diamond, B.A. et al., The New England Journal of Medicine, Seite 1344 - 1349, (1981)). Die polyklonalen Antikörper, die in dem Tier als Ergebnis einer immunologischen Reaktion gebildet werden, können nachfolgend aus dem Blut unter Verwendung von im Stand der Technik bekannten Verfahren isoliert werden und dann beispielsweise mittels Säulenchromatographie gereinigt werden. Monoklonale Antikörper können beispielsweise in Übereinstimmung mit dem bekannten Verfahren von Winter & Milstein (Winter, G. & Milstein, C., Nature, 349, Seite 293 - 299, (1991)) hergestellt werden.

Spezifische polyklonale Antikörperseren, die gegen den humanen Fn14 gerichtet sind, können unter Anwendung konventioneller Methoden hergestellt werden, indem Tiere, beispielsweise Ziegen, Kaninchen oder Mäuse, subkutan beispielsweise mit einem Fn14 abgeleitetem Protein oder Peptid oder -derivat, das das aktivierende Epitop repräsentiert, injiziert werden. Alternativ kann auch DNA-Vakzinierung angewendet werden. Das aktivierende natürliche Epitop kann dabei auch durch jedwede chemische Substanzen dargestellt werden, die eine dem natürlichen Epitop vergleichbare zu bindende Oberflächenstruktur und/oder Form und/oder Ladung aufweist.
Hierbei können auch die Immunreaktion verstärkende intraperitonale oder subkutane Injektionen von zusätzlichen Agentien (Adjuvantien, z.B. Freunds Adjuvanz) verwendet werden. Polyklonale Antiseren, die die gewünschten Antikörper enthalten, die gegen das aktivierende Epitop des Fn14 gerichtet sind, können durch intraperitonale sich wiederholende Immunisierung von Mäusen mit Fn14 abgeleitetem Protein oder Peptid oder -derivat in Abwesenheit vom Adjuvanz expandiert werden. Die Immunisierung der Tiere durch Fn14, abgeleiteten Proteinen, Peptiden oder -derivaten, welche das aktivierende Epitop repräsentieren, kann als Injektionen sowohl intraperitoneal als auch intravenös erfolgen.
Zur Herstellung von monoklonalen Antikörpern, können Hybridomazellen nach klassischen Methoden fusioniert werden und beispielsweise mittels ELISA (Ling et al., J. Interferon and Cytokine Res., 15, Seite 53 - 59 (1995)) gescreent werden. Hybridomazellen werden weiterhin auf ihre Fähigkeit, Antikörper zu produzieren, die den Fn14, abgeleitetes Protein, Peptid, -derivat oder das aktivierende Epitop erkennen, und die Fettzelldifferenzierung zu modulieren, untersucht. Reine monoklonale Antikörper (IgG) wurden von Hybridomazellkulturüberständen mittels Protein A Sepharose aufgereinigt.

Zahlreiche Formen von anti-Fn14-Antikörpern können ebenso unter Anwendung von Standardmethoden zur Produktion von rekombinanter DNA hergestellt werden (Winter und Milstein, Nature, 349, Seite 293 - 299 (1991)). Zum Beispiel können "chimäre" Antikörper, in denen die Antigenbindestelle von einem Tierantikörper mit einer humanen konstanten Domäne verknüpft ist, hergestellt werden (z.B. Cabilly et al., US 4,816,567; Morrison et al., Proc. Natl. Acad. Sci. U.S.A., 81, Seite 6851 - 6855 (1984)). Chimäre Antikörper reduzieren die beobachtete Immunantwort, was in humanen klinischen Studien deutlich wird, in denen Tierantikörper verwendet werden. Entsprechend der vorliegenden Erfindung wird deshalb der Ausdruck Antikörper und Antikörperfragment auch dahingehend verstanden, dass Antikörper und/oder Antigenbindende Teile davon eingeschlossen sind, welche rekombinant hergestellt wurden, und, wenn erforderlich, modifiziert wurden, beispielsweise chimäre Antikörper, humanisierte Antikörper, multifunktionelle Antikörper, bispezifische oder oligospezifische Antikörper, Einzelstrangantikörper und F(ab)- oder F(ab)₂-Fragmente (siehe beispielsweise EP 368 684B1, US 4,816,567, US 4,816,397, WO 88/01649, WO 93/06213 oder WO 98/24884).

Zudem können rekombinante "humanisierte Antikörper", die das aktivierende Epitop des Fn14 erkennen, synthetisiert werden. Humanisierte Antikörper sind Chimären, die zum größten Teil humane IgG-Sequenzen aufweisen, in die die für die spezifische Antigenbindung verantwortliche Regionen insertiert wurden (WO94/04679). Tiere werden mit dem gewünschten Antigen immunisiert, die entsprechenden Antikörper isoliert und der Teil der variablen Sequenzregionen, die für die spezifische Antigenbindung verantwortlich sind, entfernt. Die von den Tieren stammenden Antigenbindestellen werden dann in die entsprechende Position der humanen Antikörpergene kloniert, in denen die humane Antigenbindestellen deletiert wurden. Humanisierte Antikörper reduzieren die Verwendung von heterologen (innerartlichen) Sequenzen in humanen Antikörpern und sind weniger riskant, eine Immunantwort in dem behandelten Individuum zu induzieren.

Die Herstellung unterschiedlicher Klassen rekombinanter anti-Fn14-Antikörpern, die das aktivierende Epitop erkennen, kann auch erzielt werden, indem chimäre oder humanisierte Antikörper mit anti-Fn14-variablen Domänen und humanen konstanten Domänen (CH1, CH2, CH3) hergestellt werden, die aus unterschiedlichen Klassen von Immunglobulinen isoliert wurden. Zum Beispiel können anti-Fn14-Antikörper, die das rezeptor-aktivierende Epitop erkennen, mit einer erhöhten Affinität für Antigenbindungsstellen rekombinant hergestelt werden, indem die Antigenbindungsstellen in Vektoren kloniert werden, welche korrespondierende humane konstante Regionen enthalten (Arulandam et al., J.Exp. Med., 177, Seite 1439 - 1450 (1993); Lane et al., Eur. J. Immunol., 22, Seite 2573 - 2578 (1993); Traunecker et al., Nature, 339, Seite 68 - 70 (1989)).

Als Alternative zu klassischen Antikörpern ist es auch möglich, Proteingerüste (sogenannte "protein scaffolds") gegen das jeweilige Molekül zu verwenden, beispielsweise Anticaline, die auf Lipocalin basieren (Beste et al., Proc. Natl. Acad. Sci. USA, 96, Seite 1898-1903, (1999)). Die natürlichen Liganden-Bindungsstellen von Lipocalinen, beispielsweise von Retinol-bindenden Protein oder Bilin-bindenden Protein, können verändert werden, beispielsweise unter Verwendung eines "kombinatorischen Protein-Design"-Ansatzes, und zwar auf eine solche Weise, dass sie ausgewählte Haptene binden, (Skerra, Biochem. Biophys. Acta, 1482, Seite 337 - 350, (2000)). Von anderen Proteingerüsten ist bekannt, dass sie Alternativen für Antikörper darstellen (Skerra, J. Mol. Recognit, 13, Seite 167-287, (2000)). Alle diese Protein-abgeleiteten Alternativen zu Antikörpern können als Bindeproteine bezeichnet werden. Entsprechend der vorliegenden Erfindung wird deshalb der Ausdruck Bindeprotein dahingehend verstanden, dass die hierin beschriebenen Bindeproteine und Bindemoleküle z.B. Affiline, Anticaline oder Aptamere ebenfalls eingeschlossen sind, welche spezifisch das rezeptor-aktivierende Epitop des Rezeptors Fn14, daraus abgeleiteten Proteinen, Peptiden oder -derivaten erkennen, welche rekombinant hergestellt und, wenn erforderlich, modifiziert wurden.

### Epitope des Fn14, welche bei Bindung durch anti-Fn14-Antikörper eine Rezeptoraktivierung zur Folge haben

Antikörper binden mit definierter Spezifität und Affinität an die dazugehörigen Zielmoleküle. So auch Antikörper, die gegen Rezeptoren auf der Zelloberfläche gerichtet sind. Die Bindung eines Antikörpers an einen solchen Obertlächenrezeptor, hier Fn14, kann dessen Aktivierung bewirken. Dazu ist die blosse Bindung des Antikörpers notwendig, aber nicht hinreichend. Vielmehr ist die Rezeptoraktivierung abhängig von der Art der Bindung des Antikörpers. Diese Art der Bindung durch den Antikörper muss den Effekt simulieren, welchen die Bindung eines natürlichen Liganden an den Rezeptor hat. In vielen Fällen bewirkt der natürliche Ligand eine Änderung der Form (Konformation) oder einer anderen Eigenschaft des Rezeptors, zum Beispiel seine Dimerisierung oder Multimerisierung, oder beides. Eine Aktivierung erfolgt dann, wenn diese strukturellen Änderungen eine intrinsische Aktivität des Rezeptorproteins, zum Beispiel eine Enzymaktivität, oder eine Protein-Proteinwechselwirkungsaktivität direkt oder indirekt zur Folge haben. Die induzierten Veränderungen des Rezeptors sind abhängig von der molekularen Position oder den Positionen auf dem Rezeptor, an den/die der Ligand oder Antikörper bindet. Molekulare Positionen, welche von Antikörpern erkannt werden, werden als Epitope bezeichnet. Dementsprechend binden Antikörper, welche zur Aktivierung von Rezeptoren führen, an solche Epitope, die bei Bindung die Rezeptoreffekte der Ligandenbindung (s.o.) simulieren.

Diese Peptide, Epitope oder artifizielle chemische Strukturen, welche den Epitopen funktionell entsprechen, können sowohl zur Immunisierung von Tieren zur Generierung beispielsweise polyklonaler oder monoklonaler Antikörper als auch zur Generierung von Antikörperderivaten und Bindeproteinen mittels rekombinanter Technologien verwendet werden. Zusätzlich ist es ebenfalls möglich, diese aktivierenden Epitope als Immunogen zur Bildung von aktivierenden Antikörpern direkt im Menschen zu verwenden. Dieses ermöglicht eine Vakzinierung von Patienten, mittels derer Adipositas und deren Folgeschäden vorgebeugt und/oder behandelt werden kann.

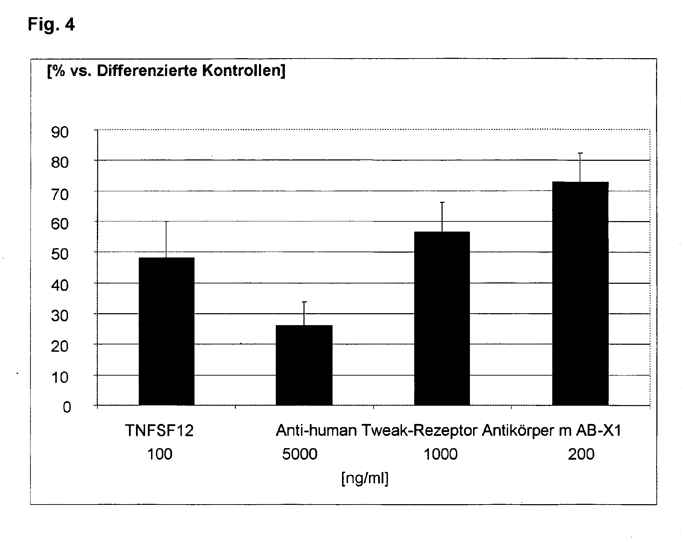

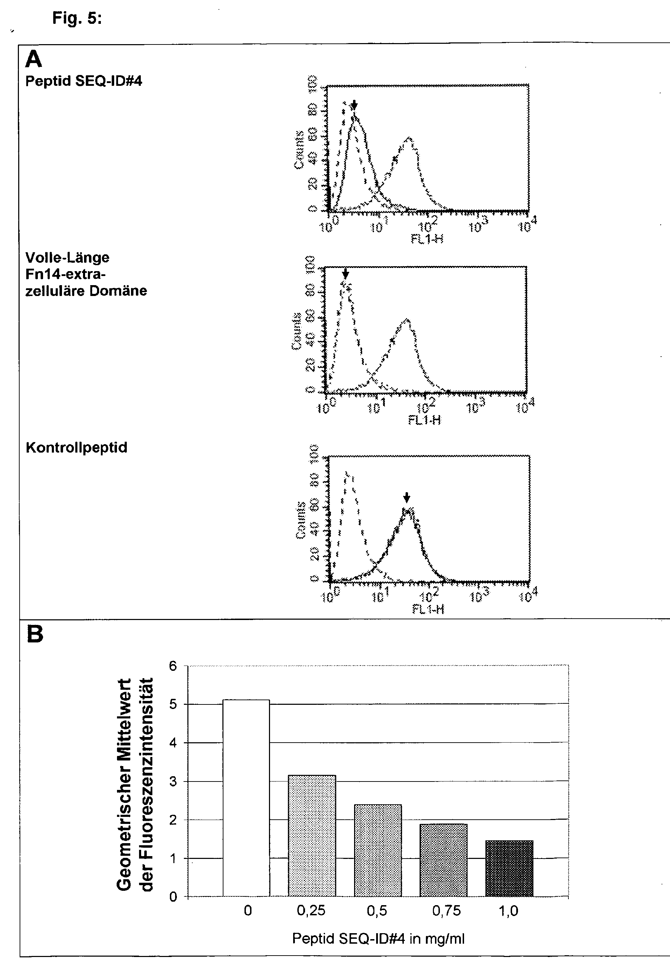

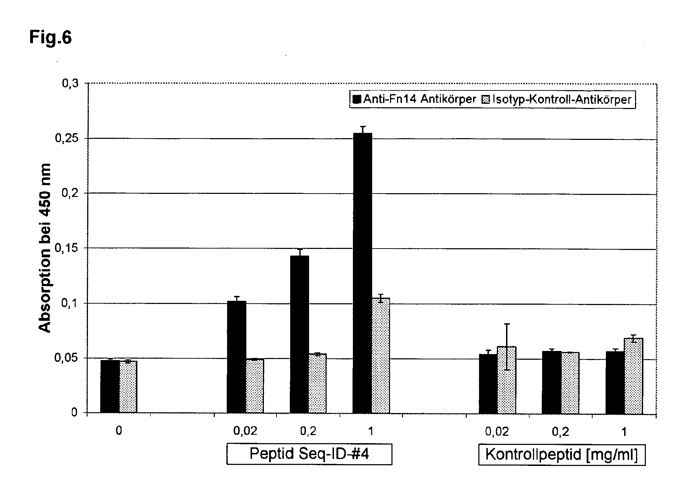

Das Wissen über die Epitope, welche bei Bindung zur Rezeptoraktivierung führen, kann unter anderem dazu verwendet werden, mit rationalen Verfahren gezielt agonistische Antikörper zu generieren. Dieses kann u.a. durch Immunisierung mit solchen Epitopen erfolgen. Andererseits können basierend auf aktivierenden Epitopen auch direkte Bindungsassays, welche beispielsweise auf dem ELISA-Prinzip beruhen (s. Beispiel 5, Figur 6) etabliert und verwendet werden. Solche Assays können direkt zur Identifizierung und Charakterisierung aktivierender Antikörper angewendet werden. (s. Beispiel 5, Figur 6).
Alternativ dazu ist es andererseits ebenfalls möglich, Antikörper, welche solche Epitope binden, auch über Kompetitionsexperimente zu erlangen. So können potentiell agonistische Antikörper über Peptid-Kompetitionen identifiziert werden. Bei einem solchen aktivierenden Antikörper handelt es sich beispielsweise um den agonistischen Antikörper mABX1 welcher in Beispiel 2 und Figur 4 als agonistischer anti-Fn14-Antikörper beschrieben ist. Kompetitionsexperimente, bei denen die zu analysierenden Antikörper daraufhin getestet werden, ob sie bei Zugabe im Überschuss eines definierten Epitopes die Bindung des zu testenden Antikörpers inhibieren, weisen auf Epitop-spezifische Bindung hin. Solche Kompetitionsanalysen, deren Durchführung von einem Fachmann mittels Standardmethoden durchführbar sind, und welche beispielhaft in Figur 5 dargestellt sind, können dementsprechend ebenfalls zur Analyse der potentiellen Bindung von neuen Antikörpern an aktivierende Epitope des Fn14 verwendet werden.

### Modulation der Fettzelldifferenzierung durch spezifische Aktivierung der Fn14-vermittelten Signaltransduktion

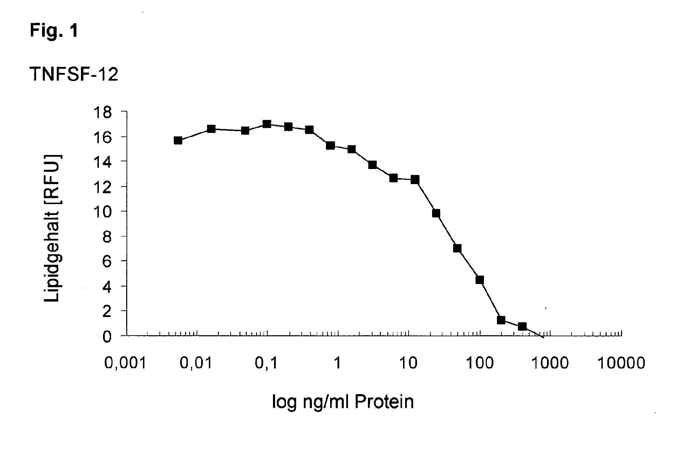

Gemäß einer bevorzugten Ausführungsform wirken die erfindungsgemäß verwendeten anti-Fn14-Antikörper, -Antikörperderivate oder -Bindeproteine dadurch, dass sie die Bildung terminal differenzierter Adipozyten hemmen. Dabei kann die Bildung und/oder Funktion terminal differenzierter Adipozyten zum einen dadurch gehemmt werden, dass die Differenzierung zu terminal differenzierten Adipozyten gehemmt wird, insbesondere durch Hemmung des Transkriptionssignalweges zur Differenzierung. Zum anderen kann die Bildung terminal differenzierter Adipozyten durch Hemmung der Lipogenese oder durch Förderung der Lipolyse gehemmt werden. Die Hemmung der Differenzierung zu terminal differenzierten Adipozyten kann durch Messung des Lipides (z.B. durch Färbung mit Nile Red) erfolgen. Mit Reportergenassays wie z.B. für PPAR-γ oder CEBP-β oder -δ lässt sich die Hemmung des Transkriptionssignalweges messen. Durch Färbung mit lipophilen Farbstoffen wie z.B. Nile Red lässt sich der Lipidgehalt in Adipozyten nachweisen. Die Modulation der Adipozytendifferenzierung durch Aktivierung von Fn14 ist sowohl für die natürlichen Rezeptor-Liganden TNFSF12, als auch für aktivierende anti-Fn14- Antikörper in Beispiel 2 beschrieben. Hierbei wird sowohl in murinen Zellen als auch in frischen menschlichen Präadipozyten der Effekt durch die Liganden- (TNFSF12) und Antikörper (anti-Fn14)-vermittelte Signaltransduktion klar und in einer dosisabhängigen Weise darstellbar (siehe Figuren 1, 2 und 4).

Weil eine Involvierung von Fn14-vermittelter Signaltransduktion in entzündliche und toxische Prozesse und Zelltod (beispielsweise in Tumorzellen) schon bekannt ist, wurde ebenfalls analysiert, ob die Hemmung der Differenzierung von Adipozyten mit Induktion vom Zelltod assoziiert ist. Die Bestimmung von Lebendzellzahlen ist beispielsweise durch ,Alamar Blue' (Biosource) oder ,CeIITiterGlo' (Promega) Assays möglich.

Die Möglichkeit von Toxizität oder Induktion von Apoptose durch Aktivierung von Fn14 wurde von der Anmelderin in murinen und menschlichen Zellen untersucht und ist in Beispiel 3 beschrieben. Es konnte gezeigt werden, dass unter den verwendeten Bedingungen keine Toxizität auftrat. In diesen Experimenten wurde ebenfalls demonstriert, dass der hemmende Effekt reversibel ist. Dieses konnte durch Wegnahme des Proteins gezeigt werden, was in Folge den Zellen einen Wiedereinstieg in den Differenzierungsprozess ermöglicht. Offensichtlich ist dieses nur lebenden Zellen möglich, was auch durch mikroskopische Messungen der lebendigen Zellen bestätigt werden konnte.

### Therapeutische Verwendung von Antikörpern und anderen Aktivatoren der Fn14-vermittelten Signaltransduktion zur Vorbeugung und/oder Behandlung von Adipositas und assoziierten Krankheiten

Die Erfindung betrifft ein Verfahren zur Behandlung eines Patienten, bei welchem dem Patienten eine wirksame Menge eines und/oder mehrerer der oben definierten agonistischen Agenzien, wie anti-Fn14-Antikörper, verabreicht wird. Bevorzugt leidet der Patient unter Adipositas. Für dieses erfindungsgemäße Verfahren gelten alle oben für die erfindungsgemäße Verwendung aufgeführten Ausführungsformen.

Wie bereits oben beschrieben, ermöglichen der Faktor TNFSF12 und die erfindungsgemäß gefundenen anti-Fn14-Antikörper die Inhibition der Differenzierung von Präadipozyten zu Adipozyten und/oder eine Hemmung der Funktion der reifen bzw. des reifenden Adipozyten beispielsweise durch die Hemmung der Lipogenese oder Förderung der Lipolyse.

Die Beobachtungen, dass gezielte Rezeptoraktivierung von Fn14 durch rekombinante Supplementation des rezeptoraktivierenden Liganden TNFSF12 das Gewicht von Tieren reduzieren kann (s. Beispiel 4), ist ein Beweis für das hier beschriebene therapeutische Konzept.

Unter Inhibieren bzw. Hemmen wird erfindungsgemäss das vollständige oder teilweise Unterdrücken einer biologischen Funktion verstanden. In unserem speziellen Fall ist die zu inhibierende Funktion die Differenzierung von Adipozyten in adipösen Patienten oder die Vorbeugung zur Verhinderung von Adipositas. Entsprechend der vorliegenden Erfindung beschreibt der Ausdruck "Inhibitor" ein Agenz, wie einen anti-Fn14-Antikörper, das vorzugsweise den Prozess der Fetteinlagerung selbst inhibiert. In diesem Fall kann der aktivierende Antikörper als Inhibitor bezeichnet werden. Die Inhibition kann beispielsweise durch Induktion von Fn14-abhängiger Signaltransduktion und darauffolgende Modulation der Expression regulierter spezifischer Gene erreicht werden.

Gemäß einer bevorzugten Ausführungsform wirkt der verwendete Inhibitor, z.B. der anti-Fn14-Antikörper, dadurch, dass er die die Bildung terminal differenzierter Adipozyten hemmt. Dies kann beispielsweise und präferentiell geschehen durch Interferenz der Differenzierung zu terminal differenzierten Adipozyten, insbesondere durch Modulation des Fn14-Transkriptionssignalweges bei der Differenzierung in Präadipozyten.

Die Erfindung betrifft ferner eine pharmazeutische Zusammensetzung, welche ein oder mehrere Agenzien der vorliegenden Erfindung enthält. Für alle pharmazeutischen Zusammensetzungen gemäß der vorliegenden Erfindung gilt das folgende:

Für die Herstellung der erfindungsgemäßen pharmazeutischen Zusammensetzung werden die entsprechenden Moleküle gewöhnlich mit geeigneten Additiva oder Hilfsstoffen formuliert, wie beispielsweise physiologische Pufferlösung, beispielsweise Natriumchloridlösung, demineralisiertes Wasser, Stabilisatoren, wie Protease- oder Nukleaseinhibitoren, vorzugsweise Aprotinin, ε-Aminocapronsäure oder Pepstatin A oder Chelatbildner wie EDTA, Gelformulierungen, wie weiße Vaseline, Paraffin mit geringer Viskosität oder Gelbwachs (yellow wax), abhängig von der Art der Verabreichung.

Geeignete zusätzliche Additiva sind beispielsweise Detergenzien, wie beispielsweise Triton X-100 oder Natriumdeoxycholat, aber auch Polyole, wie beispielsweise Polyethylenglycol oder Glycerin, Zucker, wie beispielsweise Sucrose oder Glucose, zwitterionische Verbindungen, wie beispielsweise Aminosäuren, wie Glycin oder insbesondere Taurin oder Betain oder ein Protein, wie beispielsweise bovines oder humanes Serumalbumin. Detergenzien, Polyole und/oder zwitterionische Verbindungen sind bevorzugt.

Die physiologische Pufferlösung hat vorzugsweise einen pH von etwa 6,0-8,0, insbesondere einen pH von etwa 6,8-7,8, insbesondere einen pH von etwa 7,4, und/oder eine Osmolarität von ungefähr 200-400 milliosmol/Liter, vorzugsweise von etwa 290-310 milliosmol/Liter. Der pH des Medikaments wird im Allgemeinen unter Verwendung eines geeigneten organischen oder anorganischen Puffers angepasst, wie beispielsweise unter Verwendung von Phosphatpuffern, Trispuffern (Tris(hydroxymethyl)aminomethan), HEPES-Puffer ([4-(2-Hydroxyethyl)piperazino]-ethansulfonsäure) oder MOPS-Puffer (3-Morpholino-1-propansulfonsäure). Die Wahl des entsprechenden Puffers hängt im Allgemeinen von der gewünschten Puffermolarität ab. Phosphatpuffer ist beispielsweise geeignet für Injektions- oder Infusionslösungen.

Injektionslösungen werden im allgemeinen verwendet, wenn nur eine geringe Menge einer Lösung oder Suspension, beispielsweise etwa 1 bis etwa 20 ml, verabreicht werden sollen. Infusionslösungen werden im allgemeinen verwendet, wenn eine größere Menge einer Lösung oder Suspension, beispielsweise ein oder mehrere Liter, verabreicht werden sollen. Da im Gegensatz zur Infusionslösung nur wenige Milliliter im Fall von Injektionslösungen verabreicht werden, sind geringe pH-Differenzen oder des osmotischen Drucks des Bluts oder der Gewebeflüssigkeit im Vergleich zur Injektionslösung selbst nicht bemerkbar oder spielen keine große Rolle. Die Verdünnung der Formulierung vor der Verwendung ist daher im allgemeinen nicht erforderlich. Wenn allerdings relativ große Mengen verabreicht werden, sollte die erfindungsgemäße Formulierung kurz vor der Verabreichung verdünnt werden, so dass etwa eine isotonische Lösung erhalten wird. Ein Beispiel einer isotonischen Lösung ist eine 0,9%-ige Kochsalzlösung.

Grundsätzlich wird die wirksame Dosis vom Gewicht und dem Zustand des jeweiligen zu behandelnden Subjektes abhängen. Dabei ist davon auszugehen, dass dem Fachmann bekannt ist, wie eine geeignete Dosierung bestimmt werden kann.

Die pharmazeutische Zusammensetzung kann auf verschiedene Art und Weise verabreicht werden, beispielsweise intramuskulär, subkutan, intrathekal, in das Fettgewebe, perkutan (gelöst in DMSO), intravenös oder intraperitoneal, oder durch Fusion oder Gele, welche das jeweilige Medikament enthalten. Es ist ferner möglich, das Medikament topisch und lokal zu applizieren. Ferner kann das Medikament durch ein transdermales therapeutisches System (TTS) verabreicht werden, welches eine zeitlich kontrollierte Abgabe des Medikaments ermöglicht. TTS sind beispielsweise aus EP 944398, EP 916366, EP 889723 oder EP 852493 bekannt. Eine langsamere Abgabe des Proteins, Peptids, oder Antikörpers wird durch Kombination mit Polymeren, eine längere Halbwertszeit durch Zugabe von PEG erreicht. Geeignete Polymere erlauben auch eine perorale Verabreichung der pharmazeutischen Zusammensetzung, beispielsweise durch eine geschützte Passage durch den Darm und die gezielte Penetration der Zell-Zell-Kontaktstellen im Bereich des Darmepithels. Eine Kombination mit chemischen Wirkstoffen (z.B. Appetithemmer, Lipasehemmer) kann die Wirkung noch verstärken.

Gemäß einer bevorzugten Ausführungsform liegt die pharmazeutische Zusammensetzung mit einem aktivierenden Agenz gemäß der vorliegenden Erfindung in einer Form vor, welche den Kontakt des Wirkstoffes mit einem Bestandteil der Zelloberfläche (nicht zellgängig) ermöglicht (Cardiovasc Pharmacol Ther., 7 (3) Seite 171 - 80, (Juli 2002)).

Die Aufzählungen in Gestalt der oben angeführten Erwähnungen sollen die vorliegende Erfindung verdeutlichen, jedoch nicht limitieren.

Die Erfindung betrifft ferner ein Verfahren zur Vorbeugung von Adipositas und/oder Behandlung eines Patienten, bei dem Patienten eine wirksame Menge eines oder mehrerer der oben definierten Inhibitoren verabreicht wird. Bevorzugt leidet der Patient unter Adipositas oder Diabetes Typ II- oder unter Diabetes Typ II-verwandten Krankheiten.

### Beispiele

Die Erfindung wird im folgenden durch Beispiele und Figuren erläutert, die in keiner Weise dazu gedacht sind, den Gegenstand der Erfindung einzuschränken, sondem sie stellen Ausführungsformen dar.

In den Beispielen wird insbesondere gezeigt, dass die erfindungsgemäß verwendeten löslichen Polypeptide und Antikörper in der Lage sind, die Lipideinlagerung in murinen 3T3-L1 Zellen und in menschlichen Präadipozyten zu inhibieren. Die Inhibition konnte sowohl in primären humanen Adipozyten subkutanen als auch omentalen Ursprungs gezeigt werden. Der Nachweis erfolgte jeweils durch Nile Red-Assay.

Ferner wird gezeigt, dass der Rezeptor Fn14, dessen Aktivierung die Modulation der Fettzelldifferenzierung bewirkt, auf menschlichen und murinen Fettzellvorläuferzellen vorhanden ist. Diese Tatsache ist eine wichtige Voraussetzung, um den beobachteten Phänotyp durch rezeptorvermittelte Signaltransduktion zu erklären.

Desweiteren wird gezeigt, dass die Aktivierung des Rezeptors Fn14, welche die Modulation der Fettzelldifferenzierung bewirkt, in dem hier verwandten System muriner und humaner Zellkulturen zu keinen toxischen Effekten führt.

Zusätzlich wird gezeigt, dass exprimierte lösliche murine homologe Varianten von TNFSF12 bzw. dessen Proteinfragmente in der Lage sind, die Gewichtszunahme in Mäusen zu reduzieren, ohne generelle toxische Effekte zu verursachen.

Es wird ebenfalls gezeigt, dass Antikörper, welche durch Rezeptoraktivierung den Fettzell-Modulierenden Phänotyp auslösen, definierte Epitope des Rezeptors Fn14 binden und dadurch die Signaltransduktion auslösen. Ein solches definiertes aktivierendes Epitop von Fn14 wird durch das Peptid MDCASCRARPHSDFC (vgl. SEQ ID NO 4) repräsentiert. Das Wissen um dieses Epitop ist eine wesentliche Voraussetzung für die rationale Generierung von definierten Antikörpern, welche durch gezielte Bindung an diese Epitope des Rezeptors die Signaltransduktion aktivieren.

### Beschreibung der Figuren:

Fig. 1:
   Dargestellt ist die Dosis Wirkungskurve, die den Lipidgehalt von 3T3-L1 Zellen in Abhängigkeit der zugesetzten Menge an rekombinantem sekretiertem bzw. löslichem Proteinfragment TNFSF-12 zeigt.
   Die Messergebnisse sind als relative Fluoreszenzeinheiten (RFU) am Tag 5 nach Zugabe des Proteins angegeben.
Fig. 2
   Dargestellt ist die prozentuale Hemmung der Lipideinlagerung am Tag 8 in Form einer Dosis-Wirkungskurve- durch jeweils rekombinante sekretierte bzw. lösliche Proteinfragmente von TNFSF12 - in primären humanen Adipozytenzellkulturen subkutanen Ursprungs. Dazu wurden die primären humanen Zellkulturen mit den jeweiligen rekombinanten Proteinen inkubiert. Die prozentualen Daten beziehen sich jeweils auf Kontrollkulturen die mit einer noch unwirksamen Proteinkonzentration von 0,025ng/ml behandelt wurden. Eine niedrige Signalstärke bedeutet geringe Lipideinlagerung in die differenzierenden humanen Adipozyten, eine hohe Signalstärke bedeutet eine starke Lipideinlagerung.

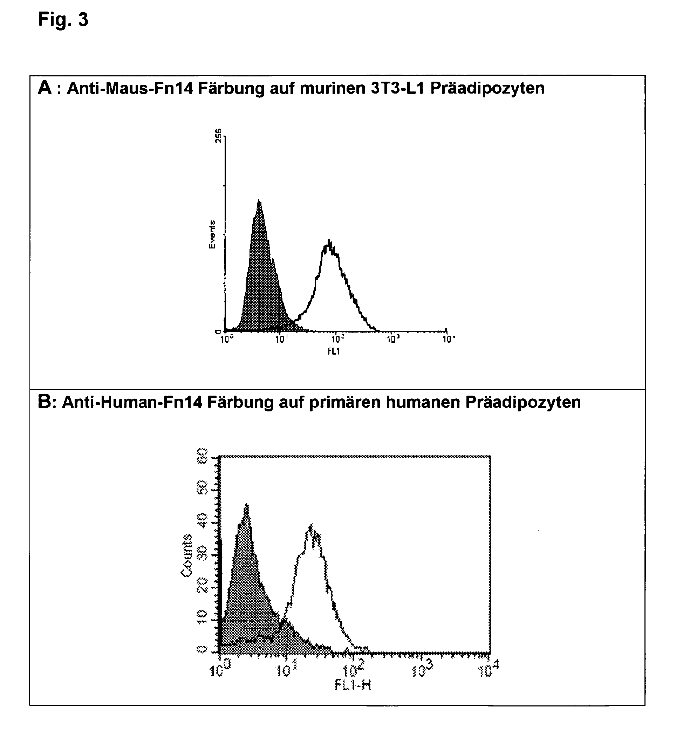

Fig. 3: Nachweis von Fn14 auf murinen und humanen Präadipozyten
   Dargestellt ist der Nachweis des Rezeptors von TNFSF12, Fn14, auf Präadipozyten. A: Maus 3T3-L1 Präadipozyten gefärbt mit anti-Maus-Fn14 spezifischem Antikörper und B: primäre humane Adipozyten gefärbt mit anti-human-Fn14 spezifischem Antikörper und je einem geeigneten Fluorophor markierten sekundären Antikörper und dem Fluoreszenz-aktivierten Zellsortierungsverfahren (FACS). Die Messergebnisse sind als Fluoreszenzintensitäten (FL-1) im Verhältnis zur Ereigniszahl ("event" bzw. "counts") angegeben. Verglichen wurde jeweils das Fluoreszenz-Intensitätsprofil in Anwesenheit (□) bzw. Abwesenheit (■) des spezifischen Rezeptor-Antikörpers. Eine Verschiebung der Fluoreszenzintensität hin zu stärkeren Intensitäten (Rechtsverschiebung), bedeutete eine Bindung des spezifischen Rezeptorantikörpers gepaart mit dem Fluoreszenz-markierten sekundären Antikörpers an die Zelloberfläche. Dies zeigt die Rezeptor-Exposition an der Zelloberfläche an.
Fig. 4:
   Dargestellt ist die konzentrationsabhängige Hemmung der Lipideinlagerung durch zugesetzten Antikörper mAB-X1 im Vergleich zu TNFSF12 in primären humanen Adipozyten subkutanen Ursprungs. Die Messergebnisse sind in Prozent versus Lipideinlagerung von normal differenzierten Zellen angegeben. Die Daten wurden am Tag 8 nach erstmaliger Zugabe des jeweiligen Proteins und der Induktion zur Differenzierung erhoben. Eine niedrige Signalstärke bedeutet geringe Lipideinlagerung in die differenzierenden humanen Adipozyten, eine hohe Signalstärke bedeutet eine starke Lipideinlagerung.
Fig. 5:
   Dargestellt ist das Ausmaß der Bindung eines Fn14-spezifischen Antikörpers mAB-X1 an den Fn14 Rezeptor auf HeLa-Zellen nach Präinkubation des Fn14 Antikörpers mit dem erfindungsgemässen Peptid mit SEQ-ID#4 mittels FACS-Analyse . HeLa Zellen exprimieren den Rezeptor Fn14 natürlicherweise, was hier zuvor durch den Nachweis der Bindung von TNFSF12 in einer vergleichbaren FACS Analyse nochmals bestätigt worden war.
   A: Dargestellt ist eine Analyse der Kompetition der Bindung des Fn14 spezifischen Antikörpers mAB-X1 durch das Peptid mit SEQ-ID#4. Zur Positiv-Kontrolle wurde als Kompetitor ein Fusionsprotein enthaltend die komplette humane Fn14 extrazellulärer Domäne sowie zur Negativ-Kontrolle ein Kontrollpeptid eingesetzt. Angegeben sind für jedes Kompetitionsexperiment jeweils die Fluoreszenzintensitätsverteilungen für HeLa Zellen, wie sie mit dem Fn14-spezifischem Antikörper (mit Kompetitor - durchgezogene Linie bzw. ohne Kompetitor - eng gestrichelte Linie) oder einem Isotypkontrollantikörper an Stelle des Fn14 spezifischen Antikörpers (gesperrt gestrichelte Linie) erhalten wurden. Die Fluoreszenzintensitätsverteilung nach Kompetitorzugabe ist jeweils zusätzlich mit einem Pfeil im Maximum markiert. Eine Linksverschiebung der Fluoreszenzverteilung mit Kompetitor (durchgezogene Linie) gegenüber der Verteilung ohne Kompetitor (eng gestrichelte Linie) zeigt dessen kompetitive Bindung an den Fn14-spezifischen Antikörper an. B: Steigende Mengen an Peptid mit SEQ-ID#4, führten zu einer dosis-abhängigen Abschwächung der auf den Zellen gemessenen Fluoreszenzintensität. Die Messergebnisse sind hier als der jeweils gemessene geometrische Mittelwert der Fluoreszenzintensitätsverteilung angegeben.
Fig. 6:
   Dargestellt ist der Nachweis der Bindung des Fn14-spezifischen Antikörpers mAB-X1 (schwarzer Balken) und zur Kontrolle eines nicht Fn14 spezifischen Isotyp-Antikörpers (graue Balken) an das in einer Vertiefung einer geeigneten 96-Loch-Platte immobilisierte Peptid nach SEQ-ID#4 und zur Kontrolle an ein immobilisiertes Kontrollpeptid gleicher Länge. Die Bindung wurde in Gegenwart verschiedener Mengen der Peptide in einem ELISA dosisabhängig gemessen. Die Ergebnisse sind als Absorption bei 450nm angegeben. Das dosisabhängige Signal mit Peptid SEQ-ID#4 ist für den Fn14 spezifischen Antikörper mAB-X1 signifikant stärker als für den Kontrollantikörper und tritt bei Benutzung eines sequenzverschiedenen Kontrollpeptids nicht auf.
Fig.7:
   Dargestellt ist die Restriktion der Körpergewichtszunahme in männlichen C57BL/6 Mäusen, 2 Wochen nach Injektion eines geeigneten Expressionsvektors, in Abhängigkeit vom Ausmaß der Expression löslichen bzw. sekretierten Proteinfragments von murinem TNFSF12 (A). Das Ausmaß der Expression der Proteinfragmente wurde mittels ELISA-Bestimmung an Serumproben bestimmt. Gezeigt ist die negative Korrelation von Proteinexpression und Körpergewichtszunahme. (B): Gezeigt sind Gewichtsmessungen an ausgewählten Fettdepots von Tieren in denen eine Expression von TNFSF12 nachweisbar war (n=5) im Vergleich zu solchen, in denen diese nicht oder nur vergleichsweise schwach nachweisbar (n=3) war. Exemplarisch untersucht wurden die beiden epidydimalen Fettdepots ("Pads") der männlichen Tiere und das perirenale und Kolon-assozüerte Fettgewebe. Dieses Ergebnis zeigt, daß an den ausgewählten Fettdepots eine Abnahme des Gewichts mit der TNFSF12-Expression korreliert.
Fig. 8:
   Dargestellt ist die insulinabhängige Glukoseaufnahme in primären humanen Adipozyten subkutanen Ursprungs. Unwirksame (10-10M) und wirksame (10-7 M) Konzentrationen an Insulin wurden eingesetzt. Wirksame Insulin-Konzentrationen bewirken eine gesteigerte zelluläre Glukoseaufnahme unter den gewählten Bedingungen (Kontrolle). Die Auswirkungen der Inkubation mit Protein TNFSF12 (100 ng/ml) sind gezeigt. TNF-alpha (50 ng/ml) diente in diesem Zusammenhang als Positivkontrolle, da seine Interferenz mit der insulinabhängigen Glukoseaufnahme in Adipozyten bereits beschrieben wurde. Die Messergebnisse sind angegeben als "insulinabhängige Aufnahme von pmol Glucose innerhalb von 20 Minuten".
Fig. 9:
   Dargestellt sind Untersuchungen zur proliferativen Aktivität bzw. Zellüberleben bei Exposition von subkonfluenten 3T3-L1 Zellkulturen mit löslichem TNFSF12. Die proliferative Aktivität bzw. Zellzahl wurde durch eine Lebend-Zellzahlbestimmung mittels des AlamarBlue Assays bestimmt und die Messergebnisse sind in relativen Fluoreszenzeinheiten (RFU) angegeben.

### Beispiel 1:

### Kultivierung von Präadipozytenzellen, ihre Induktion zur Differenzierung und die Messung intrazellulär eingelagerten Lipids.

### Für die Propagation und Analyse von murinen 3T3-L 1 Zellen galt:

3T3-L1 Zellen sind Maus-Fibroblasten Zellen embryonalen Ursprungs, die unter geeigneten Bedingungen eine terminale Differenzierung zu reifen Fettzellen (Adipozyten) durchführen können. Deshalb werden die in Proliferationskultur gehaltenen 3T3-L1 auch als Präadipozyten bezeichnet. Sie wachsen in Mono-Layern und besitzen in etwa eine Verdopplungszeit von 24 h. Die hier verwendeten 3T3-L1 Zellen stammten von der ATCC (CL-173). Da die 3T3-L1 Zellen bei Konfluenz spontan differenzieren und dabei die Eigenschaften der Kultur verändert werden, wurden die Zellen nicht bis zur Konfluenz kultiviert (max. 80%). Die Passagierung erfolgte in 2 Tagesabständen wobei dazwischen kein Mediumwechsel durchgeführt wurde. Die Zellen wurden erst ab Passage 5 und nur bis Passage 18 zum Screening verwendet. Die Erhaltungs-Kultur der 3T3-L1 Zellen erfolgte in T-75-Kulturflaschen. Zur Passagierung alle 2 Tage wurden diese mit 4,5 x 10⁵ Zellen / T-75-Flasche angeimpft. Es erfolgte kein Mediumwechsel bis zur nächsten Passage. Die Aussaat der 3T3-L1 Zellen für das Experiment erfolgte in 96 well Mikrotiterplatten: Die 3T3-L1 Zellen wurden in einer Zelldichte von 1,5x10⁴Zellen/well in 200 µl Wachstumsmedium/well ausgesät. Die Zellen wurden dann ohne Mediumwechsel für 3 Tage inkubiert und anschließend zur Differenzierung wie weiter unten beschrieben induziert und rekombinantem löslichem bzw. sekretiertem Proteinfragment bzw. Antikörper in Kontakt gebracht. Das 3T3-L1 Wachstumsmedium (GM) besaß folgende Zusammensetzung: DMEM, 10%FCS, 1% Penicillin-Streptomycin, 2% Glutamin, 1 % Na-Pyruvat). Das Differenzierungsmedium (DM) wurde grundsätzlich als 2fach-Konzentrat direkt nach Zugabe von rekombinantem Protein oder Antikörper zugegeben. Es besteht in dieser Form aus einem durch Induktionsfaktoren (200 nM Insulin, 1 mM IBMX und 2 µM Dexamethason) ergänzten 3T3-L1 Wachstumsmedium.

### Für die Isolierung von Adipozyten aus Fettgewebe und Kultivierung von primären humanen Präadipozyten galt:

Isolierung und Differenzierung von Präadipozyten erfolgte wie bei Hauner et al. beschrieben [Methods Mol Biol. 2001; 155: 239-47]. Das bei Operationen gewonnene Fettgewebe wird mechanisch von Bindegewebs- und Blutgefäßresten befreit und danach zerkleinert. Es folgte der Verdau mit 200 U/ml Collagenase NB4 (Serva E-lectrophoresis GmbH, Heidelberg) für 90 min bei 37 °C und 80 rpm in PBS mit 2 % BSA (3 ml Collagenaselösung/g Fettgwebe). Im Anschluss wurde für 10 Minuten bei 200 g zentrifugiert. Das entstandene Pellet enthielt neben den Präadipozyten auch Erythrozyten und Bindegewebsreste und wurde in Erythrozytenlysepuffer (155 mM NH₄Cl, 5.7 mM K₂HPO₄, 0.1 mM EDTA, pH 7.3) aufgenommen. Nach einer maximalen Inkubationszeit von 10 Minuten bei Raumtemperatur wurde über einen 150 µM Filter filtriert und wie oben beschrieben zentrifugiert. Da das verbleibende Pellet noch Bindegwebsreste enthielt, wurde nach Aufnahme in Präadiopozyten-Medium (DMEM/F12 (Invitrogen GmbH, Karlsruhe) supplementiert mit 8 mg/l Biotin, 4 mg/l Pantothenat, 1.79 g/l NaHCO₃ und 55 mg/l Pyruvat) erneut über eine 70 µM Filter gereinigt. Die Zellen wurden gezählt, erneut zentrifugiert und in DMEM/F12 mit 10 % FKS und 50 µg/ml Gentamycin resuspendiert. Die Aussaat erfolgte in einer Dichte von 40 000 bis 55 000 Zellen/cm². Die Zellen wurden bei 37 °C, 5 % CO₂ inkubiert.

### Induktion zur Differenzierung und Zugabe des zu testenden Überstandes bzw. des zu testenden Proteins auf 3T3L1 Zellen:

Drei Tage nach der Aussaat der 3T3-L1 Zellen wurden 140 µl Medium von den 3T3-L1 Zellen abgenommen und 50 µl Wachstumsmedium zugegeben, in dem geeignete Mengen an rekombinantem Protein (0-3 µg/ml Protein) bzw. Antikörper (zum Beispiel 0-10 µg/ml) gelöst wurden. Zusätzlich erfolgte nun die Zugabe von 100 µl 2X Differenzierungsmedium auf die 3T3-L1 Zellen gefolgt von einer Inkubation der 3T3-L1 Zellen im Brutschrank für 5 Tage. Nach dieser Zeit wurde eine Bestimmung des intrazellulär eingelagerten Lipids vorgenommen.

### Differenzierung von humanen Präadipozyten und SGBS Zellen:

Humane Präadipozyten wurden in einer Dichte von 40 000 bis 55 000 Zellen/cm² ausgesät. Für SGBS Zellen (beschrieben in Wabitsch et al., 2001, Int J Obes Relat Metab Disord. 25:8-15) lag die Zelldichte um den Faktor 10 niedriger. Nach der Aussaat verblieben die Zellen der Primärkultur für 20 Stunden, die SGBS Zellen für 3 Tage im Proliferationsmedium (Präadipozyten-Medium mit 10 % FKS und 50 µg/ml Gentamycin). Zur Induktion der Differenzierung wurden die Zellen zweimal mit PBS gewaschen und dann in serum-freiem Differenzierungsmedium inkubiert (Präadipozyten-Medium mit 66 nM Insulin, 1nM T₃, 100 nM Hydrocortison, 10 µg/ml Transferrin, 50 µg/ml Gentamycin). Für die ersten drei Tage wurden außerdem 1 µg/ml Troglitazon und 0,5 mM IBMX zugegeben. Nach dieser Zeit und im weiteren Verlauf alle drei bis vier Tage erfolgte ein Mediumwechsel mit Differenzierungsmedium. Die Zellen wurden verwendet, wenn mindestens 50% der Zellen (bezogen auf die Zellzahl) Fett eingelagert hatten.

### Messung des intrazellulär eingelagerten Lipids:

Zur quantitativen Bestimmung der Einlagerung von zellulärem Lipid wurde Nile Red-Reagenz (Molecular Probes, Leiden, Niederlande; CAS Nummer 7385-67-3) verwendet (Nile Red-Färbelösung: 4 µg/ml Nile Red in PBS / 40 % DMSO). Die Fluoreszenz wurde bei einer Exzitations-Wellenlänge von 485nm und einer Emissions-Wellenlänge von 590 nm gemessen. Die erforderliche Menge Nile Red wurde zur berechneten Menge DMSO zugegeben und gemischt. Anschließend wird die berechnete Menge PBS zugegeben und die Lösung gemischt. Zur Durchführung des Nile Red-Assay wurde am Tag 5 (3T3-L1 Zellen) bzw. Tag 8-12 (primäre humane Adipozytenkulturen) nach der Induktion zur Differenzierung wurde 140 µl Medium von den 3T3-L1 Zellkulturen abgenommen und 200 µl PBS zugegeben. Danach wurde 150 µl Flüssigkeit abgenommen und je 50 µl Nile Red-Färbe-Lösung zugegeben. Die Inkubation der Platten erfolgte für 4h bei 37°C im CO₂-Inkubator. Das Auslesen erfolgte in einem Fluoreszenz-Reader bei einer Exzitation von 485nm und einer Emission von 590 nm (200 msec Lesezeit). Die Messergebnisse sind als relative Fluoreszenzeinheiten (RFU) oder prozentual im Vergleich zur normal differenzierten (nicht inhibierten) Kontrolle angegeben.

### Beispiel 2:

### Inhibition der Lipideinlagerung in Zellkulturen mittels Aktivierung des Rezeptors durch rezeptorspezifische Antikörper bzw. naturgemässe Liganden.

Die Aktivierung eines zellulären Rezeptors kann durch den naturgemässen Liganden, als auch durch einen Antikörper bewerkstelligt werden, wenn die Aktivierung desselben zum Beispiel durch eine erfolgreiche Klusterbildung von Rezeptormolekülen wie im vorliegenden Fall bewirkt wird. Der Antikörper wirkt agonistisch, wenn er nach Bindung an seine Zielstruktur zu einer vergleichbaren Aktivität führt, wie sie der naturgemässe Ligand erzeugt. Dabei kann die Affinität abweichend sein. Das Epitop des Antikörpers kann, muss aber nicht zwingend der Bindungsstelle für den Liganden entsprechen. Entscheidend ist die funktionelle Wirkung auf den Aktivierungszustand des Rezeptors. Nicht jeder beliebige bindende Antikörper wird also eine Aktivierung des Rezeptors zur Folge haben, sondern nur solche, die an bestimmte Epitope in den extrazellulären Domänen des Rezeptors binden, die eine solche Aktivierung zur Folge haben.

Zunächst wurde ein Rezeptor durch seinen naturgemässen Liganden aktiviert. Dabei war im vorliegenden Fall der humane Ligand in der Lage auch den entsprechenden homologen Maus-Rezeptor zu aktivieren.

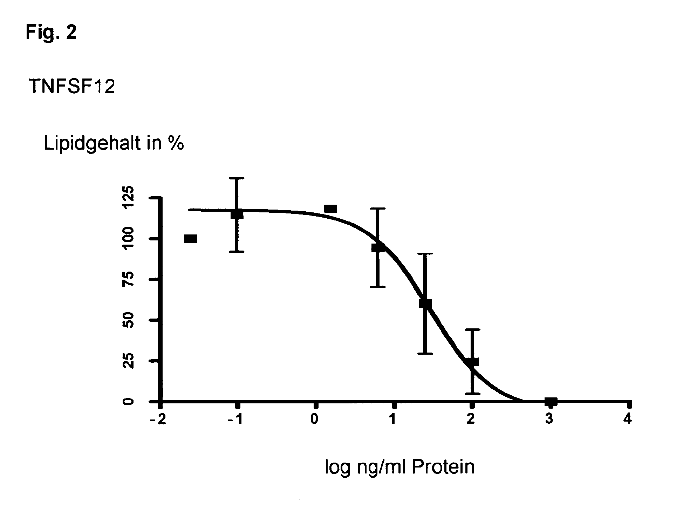

Die Hemmung der Lipideinlagerung in Maus 3T3-L1 Zellen (Fig.1) bzw. in primären humanen Adipozyten (Fig.2) wurde mit einem rekombinanten, sekretierten bzw. löslichen Proteinfragment von humanem TNFSF12 (R&D Systems GmbH, Wiesbaden; KatNo.: 1090-TW; Aminoterminal 6X HIS-getaggte extrazellulärer Domaine von humanem TWEAK (Arg 93-His 249; s. Chicheportiche, et al., 1997, J.Biol. Chem. 272:32401-32410)) bestimmt.

Die Ergebnisse zeigen, dass die Lipideinlagerung von murinen 3T3-L1 Zellkulturen (Fig.1) sowie in primären humanen Adipozyten (Fig.2) während der Differenzierung durch die Zugabe von rekombinant löslichem Proteinfragment von TNFSF12 dosisabhängig inhibiert werden kann. Die Lipideinlagerung kann als Maß für die Differenzierung der Adipozyten dienen, bzw. als ein Maß für die Regulation der Lipogenese oder Lipolyse in diesen Zellen. Es ergab sich in beiden zellulären Systemen eine halbmaximale Wirkkonzentration von etwa 30 ng/ml für das rekombinante lösliche Proteinfragment von TNFSF12. Eine ebenfalls derart vergleichbare Inhibition der Differenzierung wurde auch auf primären Fettzellen omentalen Ursprungs und der humanen SGBS Adipozytenzelllinie erreicht.
Der Einsatz eines agonistischen monoklonalen Maus Antikörpers mit Spezifität für das humane Tweak-Rezeptorprotein (Fn14) führte zu folgenden Ergebnissen:
Die Hemmung der Lipideinlagerung in primären humanen Adipozyten wurde mit dem Antikörper mAB-X1 (Fig.4) bestimmt. Die Ergebnisse zeigen, dass die Lipideinlagerung in primären humanen Adipozyten (Fig.4) während der Differenzierung durch die Zugabe von Antikörper mAB-X1 dosisabhängig inhibiert werden kann. Die Lipideinlagerung kann als Maß für die Differenzierung der Adipozyten dienen, bzw. als ein Maß für die Regulation der Lipogenese oder Lipolyse in diesen Zellen. Die Zugabe von 1 µg/ml mAB-X1 Antikörper führte in diesem Experiment zu einer Inhibition der Differenzierung, die der vergleichbar war, wenn 100 ng/ml naturgemässer Ligand eingesetzt wurden.

Um den bekannten Rezeptor von TNFSF12 auf den murinen 3T3-L1 Zellkulturen sowie auf primären humanen Präadipozyten nachzuweisen wurden diese mit jeweils spezies-spezifischen anti-Fn14-Antikörpern mittels FACS-Verfahren nachgewiesen. Dieses ist in Figur 3 dargestellt. Dabei wurde der Rezeptor von TNFSF12 mit dem Antikörperpaar aus TWEAK-Rezeptor- spezifischem Maus- (A) bzw. Human (B) Antikörper nachgewiesen. Die Färbung der Zellen erfolgte nach folgendem Schema: Aussaat von 200.000 Zellen in einer 6-well Schale. 24 Stunden später Färbung und Messung am FACS-Messgerät: Dazu wurden die Zellen trypsinisiert und pelletiert und anschliessend in 200 µl Pufferlösung (PBS + 1% FCS) bzw. Pufferlösung mit Rezeptor-spezifischen Antikörper nach Herstellerangaben (5 µg/ml) für eine Stunde auf Eis inkubiert. Danach erfolgte eine Zugabe von 1ml PBS + 1%FCS und die Pelletierung der Zellen. Wiederaufnahme erfolgte in 200 µl PBS-Puffer mit 1% FCS in dem der sekundäre Antikörper (2µg/ml) gelöst war. Es schloss sich eine weitere Inkubation auf Eis für weitere 45 Minuten an. Die Zellen wurden anschliessend wieder mit 1ml PBS Puffer+1% FCS versetzt und pelletiert. Nach Wiederaufnahme in 500 µl Puffer wurden die Zellen anschliessend der Messung im FACS Gerät zugeführt. Dabei wurde die Messung der Fluoreszenzintensitäten nur für lebende Zellen durchgeführt. Das Ergebnis zeigt, dass der für TNFSF12 beschriebene Rezeptor sowohl auf der Zelloberfläche der 3T3-L1 Zellen als auch auf der Oberfläche von primären humanen Präadipozyten exprimiert wird.

### Beispiel 3:

### Untersuchungen zu möglichen adversen Effekten nach einer Rezeptoraktivierung

Die Art und Weise, wie die erfindungsgemässen Proteine die Hemmung der Lipideinlagerung in Adipozyten bewerkstelligen, kann durch weitere Analysen in den Zellkulturen näher bestimmt werden. Eine Inhibition der Differenzierung bzw. der Lipideinlagerung in differenzierenden Adipozyten kann beispielsweise durch proteinvermittelte Aktivitäten wie Zelltod, Verhinderung des Ausstiegs aus dem Zellzyklus, Blockade eines differenzierungsspezischen Genexpression oder eine Blockade der Lipogenese im engeren Sinne oder durch eine Verstärkung der Lipolyse herbeigeführt werden.

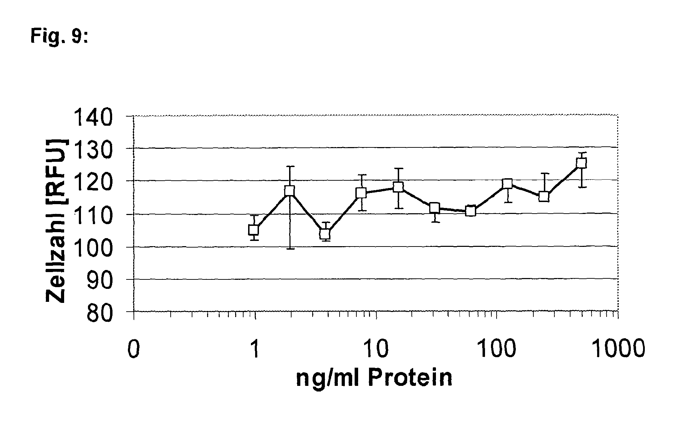

Insbesondere die Bestimmung bzw. der Ausschluss zytotoxischer Mechanismen bei der beobachteten Adipozytendifferenzierungsinhibition wurde wie folgt untersucht: Figur 9 zeigt die Bestimmung der Zellzahl von subkonfluenten 3T3-Zellkulturen. Die Zellen wurden in einer Dichte von 3.000 Zellen pro Vertiefung einer 96-well Zellkulturschale ausgesät. 24 Stunden später wurde das Wachstumsmedium der 3T3-L1 Zellen gegen Medium mit reduziertem FCS-Gehalt (0,5%) ausgewechselt. Weitere 48 Stunden später wurde ein weiterer Mediumwechsel zu 2% FCS und je zusätzlicher Gabe von löslichem rekombinantem TNFSF12 durchgeführt. Dabei wurde eine Dosis-Wirkungsbeziehung über einen Konzentrationsbereich von 0,1 bis 500 ng/ml Protein untersucht. 72 Stunden nach Zugabe der Proteine in Medium mit 2% FCS wurden zu 100 µl Kulturmedium 10 µl AlamarBlue Reagenz (Fa. BioSource) zugefügt und für 4 Stunden bei 37°C inkubiert. Das AlamarBlue Reagenz stellt ein Substrat für lebende Zellen zur Verfügung, das enzymatisch umgesetzt werden kann und das Produkt kann fluorimetrisch bei einer Exzisionswellenlänge von 530 nm und einer Emissionswellenlänge von 590 nm gemessen werden. Damit ist im linearen Messbereich eine indirekte Zellzahlbestimmung gegeben. Die Ergebnisse zeigen, dass subkonfluente 3T3-L1 Zellkulturen, durch die Zugabe von rekombinant löslichem Proteinfragment von TNFSF12 (Fig. 9) weder zu einer verstärkten Proliferation angeregt wurden, noch ein Zelltod messbar war. Diese Ergebnisse wurden auch an differenzierenden 3T3-L1 Zellkulturen bestätigt.

Zur Bestimmung der akuten *in vivo*-Toxizität einer Rezeptoraktivierung kann folgendes untersucht werden: In Mäusen des Stammes C57/B16/J wird nach Bestimmung der maximal tolerierten Dosis (MTD) a) die Verringerung der Gewichtszunahme in normalgewichtigen Mäusen (ohne vorhergehende Hochkaloriendiät) und b) die Nettoreduktion des adipösen Gewebes in zu Versuchsbeginn normalgewichtigen oder fettleibigen Mäusen (unter einsetzender oder aufrechterhaltener Hochkaloriendiät) nach Zugabe des erfindungsgemäß eingesetzten Proteins durch Wiegen oder Bestimmung des BMI ("body mass index") ermittelt. Die Bestimmung der maximal tolerierten Dosis erfolgt mit 3 Mäusen (Stamm C57B16/J Wildtyp) durch Gabe von 0,002; 0,02; 0,2; 2,0 und 10 mg Protein/kg als einmalige intravenöse Dosis. Eine anschließende Feinjustierung der Dosis erfolgt mit je 3 Mäusen (Stamm C57B16/J Wildtyp) durch einmalige Gabe des Proteins. In einem Dosistoleranztest wird durch wiederholte Gabe einer ausgewählten Dosis über mehrere Applikationen anhand von Blutparametern und Serumstabilität die zur Applikation notwendige Dosis ermittelt.
Insbesondere wurde hier bereits gezeigt, dass die einmalige intravenöse Gabe in weibliche C57B16 Mäuse von bis zu 5,4 mg/kg Körpergewicht im Falle von rekombinantem löslichem Proteinfragment von TNFSF12 zu keinerlei von der Testsubstanz verursachten klinischen Symptomen innerhalb von 48 Stunden geführt hat.

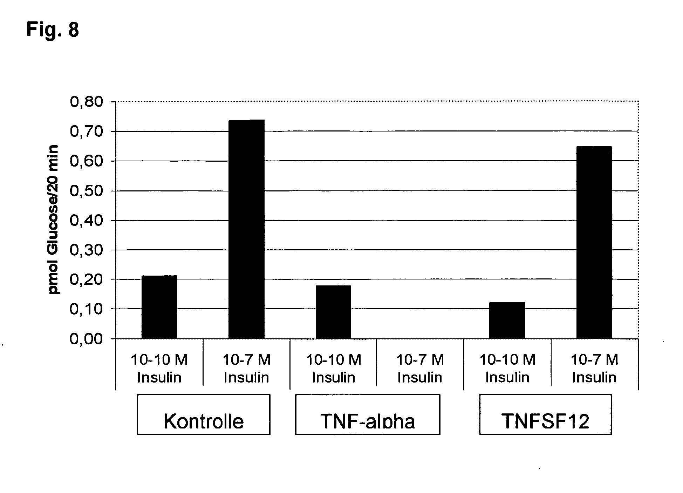

Da die Adipozytendifferenzierung ein unter anderem insulin-abhängiger Prozess ist, sollte bestimmt werden, ob die Rezeptoraktivierung zu einer negativen Modulation der Insulin-Signalgebung in Adipozyten führen kann. In diesen Experimenten, welche in Figur 8 gezeigt sind, wurden Untersuchungen zur Interferenz von TNFSF12 mit insulin-abhängiger Glukoseaufnahme in 3T3-L1 Zellen durchgeführt. 500.000 primäre Adipozyten (im Falle von FGF-16 50.000 SGBS Zellen) am Tag 12 nach der Induktion zur Differenzierung wurden in einer 3 cm Schale vorgehalten und wie folgt zur Bestimmung der insulinabhängigen Glukoseaufnahme behandelt:
Versuchsansätze: Kontrollen: 1 Zellkultur für Hintergrund (0' Insulinstimulation), Ein leeres weil/ Schälchen zur Bestimmung des Hintergrundes. Die Stimulation erfolgt mit 10⁻⁷, (ohne oder 10⁻¹⁰ Insulin, da 10⁻¹⁰ Insulin in diesem Zusammenhang nicht stimulierend wirkte. In jeder 3cm Schale befanden sich 800 µl Medium DMEM/Ham F12 mit 5 mM Glucose Differenzierte Zellen wurden vier mal mit je 2 ml PBS warm waschen, dann je Schale 800 µl DMEM/F12 mit 5mM Glukose und jeweils die erfindungsgemässen Proteine zugegeben. Anschliessend erfolgte eine Inkubation für 1 Stunde bei 37 °C. Anschliessend Zugabe von Insulin. Platten wurden bei geringster Schüttelfrequenz 15 Minuten bei 37 °C inkubiert.
2-Deoxy-D-[1-H3]-Glucose (AmershamTRK 383) wurde 1:10 verdünnt in DMEM/F12. Während der Inkubationszeit wurden Röhrchen für den Szintillationszähler bereitgestellt: eines je Messpunkt und zusätzlich eines für das leere well und eines für komplette eingesetzte Menge 2-Deoxy-D-[1-H3]-Glucose. Nach 15 Minuten Inkubation wurden zu jedem well 8 µl der verdünnten 2-Deoxy-D-[1-H3]-Glucose gegeben. Platten wurden erneut für 20 Minuten unter leichtem Schütteln bei 37 °C inkubiert. Platten wurden nach 20 Minuten auf Eis gestellt. Medium wurde mit der Pumpe abgesaugt. Zellen 3 mal mit je 1 ml PBS eiskalt gewaschen. Je well 500 µl IGEPAL (Sigma I 3021) zugegeben, 20 min auf Eis lysiert. Komplettes Volumen aus jedem Well wurde in die Szinitillationscups pipettiert. Zusätzlich in ein Cup 500 µl IGEPAL plus 8 µl der verdünnten 2-Deoxy-D-[1-H3]-Glucose. In jedes Cup 3.5 ml der Szinitillationsflüssigkeit (Rotiszint eco plus, Roth, 0016.2) pipettieren. Messung im Szintillograph über den Bereich einer Minute pro Probe. Auswertung mit Hilfe des Programms Mikro Beta Windows. In der Darstellung der Ergebnisse ist die basale Glukoseaufnahme subtrahiert, so dass nur der insulinabhängige Anteil der Glukoseaufnahme gezeigt ist.

Die Rezeptoraktivierung durch TNFSF12 zeigte in diesem Versuchsaufbau keine negative Beeinflussung der insulin-abhängigen Glukoseaufnahme.

### Beispiel 4:

### Verringerung des Gewichts bzw. der Gewichtszunahme im Mausmodell

Bevorzugte Tiermodelle sind solche, die der humanen Situation der Adipositas bzw. des Typ II Diabetes in besonderer Weise entsprechen. Dafür kommen bevorzugt Nager wie zum Beispiel Mäuse oder Ratten sowie Hunde, Schweine und Primaten in Betracht. Eine besonders bevorzugte experimentelle Ausführungsform ist eine diätinduzierte Adipositas, gegebenenfalls assoziiert mit einer Insulin-Resistenz oder Typ II-Diabetes, in Mäusen oder Ratten durch Zuführung hochkalorischer Kost. Die hochkalorische Kost, das ist zum Beispiel eine 20-45 kcal%ige Fettdiät, kann dabei bereits deutlich vor oder zeitgleich mit der Gabe der erfindungsgemässen Proteine oder Fragmente bzw. der pharmazeutischen Zusammensetzung verabreicht werden.
Die Tiere können demnach zum Zeitpunkt der Gabe des Proteins bzw. Proteinfragments normal- oder bereits übergewichtig sein. Ziel ist es eine Verringerung bzw. Verhinderung einer Gewichtszunahme und/oder eine Gewichtsreduktion zu bewirken. Gegebenenfalls auch eine Verbesserung des Insulin-Sensitivität bzw. der Glukosetoleranz. Dies kann jeweils entweder unter einsetzender oder fortgesetzter hochkalorischer Diät oder unter einem Wechsel zu normal- oder hypokalorischer Diät - für den Fall bereits übergewichtiger Tiere - erfolgen. Der Wechsel der Diäten erfolgt dabei jeweils zum Zeitpunkt der erstmaligen Gabe des Proteins bzw. Proteinfragments bzw. der pharmazeutischen Zusammensetzung.

In Mäusen des Stammes C57/B16/J wird nach Bestimmung der maximal tolerierten Dosis (MTD) a) die Verringerung der Gewichtszunahme in normalgewichtigen Mäusen (ohne vorhergehende Hochkaloriendiät) und b) die Nettoreduktion des adipösen Gewebes in zu Versuchsbeginn normalgewichtigen oder fettleibigen Mäusen (unter einsetzender oder aufrechterhaltener Hochkaloriendiät) nach Zugabe des erfindungsgemäß eingesetzten Proteins durch Wiegen oder Bestimmung des BMI ("body mass index") ermittelt.
Die Bestimmung der maximal tolerierten Dosis erfolgt mit 3 Mäusen (Stamm C57B16/J Wildtyp) durch Gabe von 0,002; 0,02; 0,2; 2,0 und 10 mg Protein/kg als einmalige intravenöse Dosis. Eine anschließende Feinjustierung der Dosis erfolgt mit je 3 Mäusen (Stamm C57B16/J Wildtyp) durch einmalige Gabe des Proteins. In einem Dosistoleranztest wird durch wiederholte Gabe einer ausgewählten Dosis über mehrere Applikationen anhand von Blutparametern und Serumstabilität die zur Applikation notwendige Dosis ermittelt.

Die applizierten Antikörper sind prinzipiell im Blutserum mittels ELISA nachweisbar. Dabei kann ein Absinken der Menge an detektierbarem Proteinfragment aus dem Blutserum über die Zeit angenommen werden, wie es für ein sich im Organismus verteilendes Protein zu erwarten ist.
Die inhibitorische Aktivität kann in den Mausseren mittels Zugabe in den 3T3-L1 Zellkulturassay mit Nile Red-Lipidbestimmung gemessen werden. Es kann so gezeigt werden, dass die Antikörper intravenös verabreicht werden können und keine klinische Toxizität verursachen.

Im Rahmen weiterführender Experimente wird die oben gefundene Maximaldosis bis zu einer geringsten Dosis verringert, die noch einen therapeutischen Effekt bewirkt. In dem anschließenden Test zur Ermittlung der Gewichtsveränderung wird die so ermittelte Dosis parenteral (intravenös, intraperitoneal, subkutan oder intramuskulär) verabreicht. Bevorzugt sind die intraperitoneale, intravenöse oder subkutane Applikation)

Zur Ermittlung der Verringerung der Gewichtszunahme in normalgewichtigen Mäusen werden normalgewichtige Mäuse unter Normalkaloriendiät (maximal 20 kcal% Fettdiät) oder insbesondere unter hochkalorischer Diät (45% kcal%) jeweils ohne (unbehandelt) und mit Zugabe des erfindungsgemäßen Proteins (behandelt) 10-14 Wochen gehalten. Während und nach Ablauf der Versuchsreihe wird die Gewichtszunahme durch Wiegen und/oder Bestimmung des BMI ermittelt.

Bei behandelten Mäusen, die unter Normalkaloriendiät gehalten wurden, ist im Gegensatz zu unbehandelten Mäusen eine Gewichtsreduktion oder verringerte Gewichtszunahme zu beobachten. Bei behandelten Mäusen, die unter Hochkaloriendiät gehalten wurden, ist im Vergleich zu unbehandelten Mäusen ein geringerer Gewichtszuwachs bzw. eine Gewichtsreduktion zu beobachten. Diese Gewichtsreduktion kann auch auf eine verringerte Fettgewebsmasse zurückgeführt werden.

Zur Ermittlung der Verringerung der Gewichtszunahme in fettleibigen Mäusen werden fettleibige Mäuse (die zuvor unter Hochkaloriendiät (45 kcal% Fettdiät) gehalten wurden), unter Normalkaloriendiät (maximal 20 kcal% Fettdiät) und/ oder unter fortgesetzter Hochkaloriendiät jeweils ohne (unbehandelt) und mit Zugabe des erfindungsgemäßen Proteins (behandelt) 10-14 Wochen gehalten. Während und nach Ablauf der Versuchsreihe wird die Gewichtszunahme durch Wiegen und/oder Bestimmung des BMI ermittelt.

Bei behandelten Mäusen, die unter Normalkaloriendiät oder fortgesetzter Hochkaloriendiät gehalten wurden, kann eine stärkere Gewichtsreduktion gezeigt werden als bei unbehandelten Mäusen. Bei unbehandelten Mäusen unter Hochkaloriendiät ist eine weitere Gewichtszunahme zu beobachten. Bei behandelten Mäusen ist hingegen eine Gewichtsabnahme bzw. eine verringerte Gewichtszunahme festzustellen.
Insbesondere wurde hier bereits in einem Tierexperiment folgendes gezeigt: Je acht fünf Wochen alte männliche C57BL6/J Mäuse wurden jeweils mit einem Expressionskonstrukt für rekombinantes sekretiertes und lösliches murines Proteinfragment von TNFSF12 injiziert. Zweck dieses Versuchsaufbaues war es, einen Einfluss der Proteinexpression auf die Zunahme an Körpergewicht zu beobachten. Die Tiere wurden über den gesamten Beobachtungszeitraum unter normal-kalorischer Kost gehalten.
Es wurde ein TNFSF12-Expressionskonstrukt verwendet, welches ein Fragment des Maus-Homologs enthielt, wie nachfolgend beschrieben. Dieses Fragment kodierte für eine rekombinante lösliche sekretierte murine Form von TNFSF12.
Die Expressionskonstrukte wurden erstellt, indem eine geeignete murine RNA-Quelle (TNFSF12: RNA aus 3T3-L1 Zellen) mit geeigneten Primern (Umfassung der Sequenzen kodierend für: Maus-TNFSF12: Arg105 - His249) eine RT-PCR durchgeführt wurde. Dabei wurden zusätzlich am 5'-Ende 6 Histidinreste und am 3'-Ende 2xTGA-Stop Codons und Sequenzen für die Restriktionsspaltstellen Xhol und BgIII eingeführt.

Verwendete Primer:
m12-F-His: 5'-CATCACCATCACCATCACCGAGCTATTGCAGCCCATTATG - 3' (vgl. SEQ-ID#1)
m12-R: 5'-AGATCTCGAGTCATCAGTGAACTTGAAAGA-3' (vgl. SEQ-ID#2)

Das Reaktionsprodukt wurde direkt in den Vektor pSECTag/FRT/V5-His-TOPO (Invitrogen, Karlsruhe, Deutschland) nach Angaben des Herstellers für die Klonierung von PCR-Produkten eingefügt. Auf diese Weise erhielt das Proteinfragment von TNFSF12 aminoterminal im Leserahmen das Sekretionssignal aus dem Protein für die Ig-kappa-Kette gefolgt von 6 Histidinresten vorangestellt. Somit kodierte der derart generierte offene Leserahmen für die folgenden sekretierten Proteine:
Maus-TNFSF12-Leserahmen (vgl. SEQ-ID#3):
METDTLLLWVLLLWVPGSTGDAAQPARRARRTKLALHHHHHHRAIAAHY-
EVHPRPGQDGAQAGVDGTVSGWEETKINSSSPLRYDRQIGEFTVIRAGLYY-
LYCQVHFDEGKAVYLKLDLLVNGVLALRCLEEFSA-
TAASSPGPQLRLCQVSGLLPLRPGSSLRIRTLPWAHLKAAPFLTYFGLFQVH

Nachfolgend wurde der Leserahmen in den Zielvektor pBS-HCRHPI-A (Miao et al, 2003, Human Gene Therapy 14:1297-1305), bestimmt für die in-vivo Genexpression in Tieren, umkloniert: Die Exzision aus pSECTag/FRT/V5-His-TOPO-Vektoren erfolgte mit Hilfe der Restriktionsspaltstellen NheI und BglII und Auffüllung der überstehenden Enden - Einfügen in pBS-HCRHPI-A erfolgte über Öffnung mit dem Restriktionsenzym EcoRV und Verbinden mit den glatten Enden mit den jeweils eingefügten Fragmenten. Das resultierende Plasmid erhielt die Bezeichnungen pXAP01 (mTNFSF12).

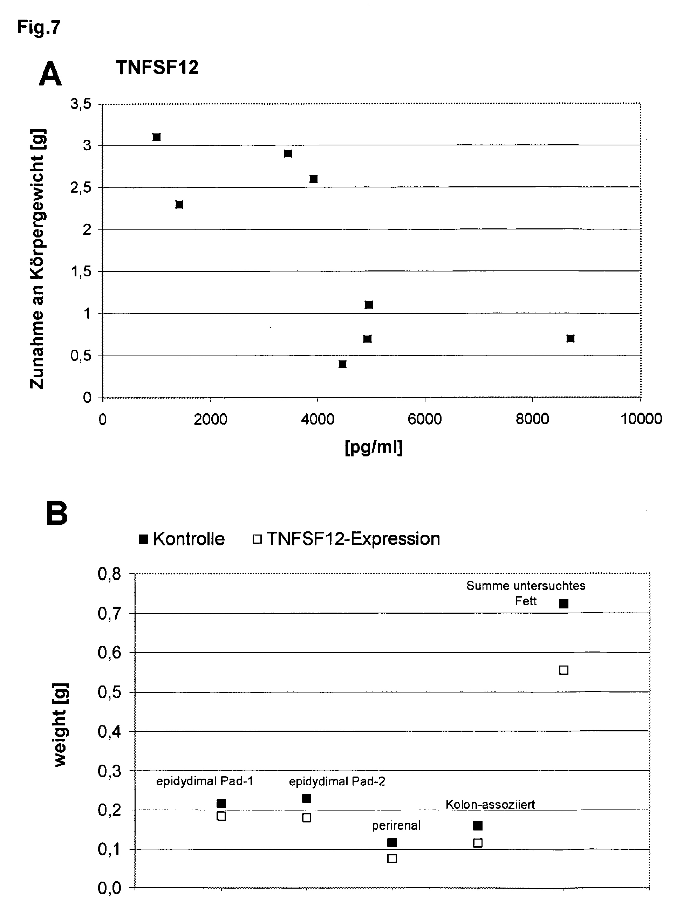

Transfektionsprozedur: Die Transfektion der Plasmide in C57BI6/J Mäuse, kodierend für rekombinantes sekretiertes und lösliches murines Proteinfragment von TNFSF12, erfolgte nach einem Verfahren, erstmals beschrieben von Zhang et al. (Gene Therapy 2000; 7:1344). Dabei wird durch einen hydrodynamischen Druck, erzeugt durch schnelle Injektion der plasmidhaltigen Lösung in die Schwanzvene, der Eintritt der Plasmid-DNA bevorzugt in Lebergewebe erreicht. Dort kann dann eine wochen- bis monatelange Genexpression realisiert werden. Das Verfahren in Kürze: Jeweils 50 µg endotoxinfrei-präpariertes Plasmid wurde in PBS-Lösung in einer Lösungsmittelmenge entsprechend jeweils 10% des Körpergewichts der zu injezierenden Maus mit einer Geschwindigkeit von 1ml/5 sec verabreicht. In der Folge wurden die Tiere bis 8 Wochen nach Injektion wöchentlich gewogen. An entnommenen Blutseren wurde der Gehalt an jeweils exprimiertem sekretierten löslichen murinen Proteinfragment von TNFSF12 mittels ELISA bestimmt. ELISA-Antikörperpaare für murines TNFSF12: Beschichtung: anti-mTWEAK-Antibody (R&D Systems, Wiesbaden Deutschland Katalognummer # AF1237), Detektions-Antikörper anti-mTWEAK-Antibody Biotinconjugated (e-bioscience, Klon MTW-1, Katalognummer #13-9913). Die Korrelation von Proteinexpression und Körpergewicht wurde untersucht.
Die Experimente beschrieben in Fig. 7 haben gezeigt, dass das Ausmaß der Expression, von murinem rekombinanten sekretierten löslichen Proteinfragment von TNFSF12, mit der beobachteten Gewichtszunahme im Untersuchungszeitraum eindeutig negativ korreliert. Es ergibt sich aufgrund der vorliegenden Ergebnisse ein Korrelationskoeffizient für die Daten aus (Fig.7A) für TNFSF12 von -0,72. Der Korrelationskoeffizient gibt an, wie strikt eine Korrelation ist. Werte zwischen -1 und +1 sind möglich. Ein Wert von 0 besagt, dass keine Korrelation vorliegt. +1 bzw. -1 sagen aus, dass eine absolut strikte (bei Vorzeichen Minus: negative) Korrelation vorliegt. Diese Korrelationen waren jeweils über den gesamten Beobachtungszeitraum von 8 Wochen zu beobachten.

Der durch die Korrelation angegebene Trend konnte für TNFSF12 auch in Wiegungen von ausgewählten Fettgewebsdepots ("epidydimale fat pads" - definierte gut messbare Fettgewebe an den Gonaden männlicher Tiere, perirenales und kolon-assoziiertes Fettgewebe) beobachtet werden (Fig. 7). Die Reduktion des Körpergewichts fand hier eine Entsprechung in einer Reduktion des Gewichts ausgewählter Fettdepots.
Es ist daher offenbar, dass weitere experimentelle Versuchsreihen, insbesondere an größeren Tierzahlen, einen gewichtsreduzierenden Effekt bestätigen werden und dieser auch auf eine Begrenzung der Ausmasse und Gewichte von Fettgewebsdepots zurückzuführen sein kann.

Solche weiterführenden Experimente sind bevorzugt, in denen zum Zeitpunkt der Inhibitorgabe 12 Wochen alte C57BI6 Mäuse bis zum Zeitpunkt der Injektion mit normal kalorischer Diät oder hochkalorischer Diät und anschliessend unter hochkalorischer Diät für weitere 8 Wochen gehalten werden. Unter diesen Bedingungen einer Diätinduzierten Adipositas ist zu erwarten, dass die Präsenz der hier beschriebenen Proteinfragmente von TNFSF12 zu einer signifikant verminderten Gewichtszunahme oder auch absoluten Gewichtsreduktion führt, bevorzugt verursacht durch ein signifikant vermindertes Gewicht der Fettgewebsdepots.

Solche weiterführenden Experimente sind bevorzugt, in denen zum Zeitpunkt der Inhibitorgabe 12 Wochen alte C57BI6 Mäuse bis zum Zeitpunkt der Injektion mit normal kalorischer Diät oder hochkalorischer Diät und anschliessend unter hochkalorischer Diät für weitere 8 Wochen gehalten werden. Unter diesen Bedingungen einer Diätinduzierten Adipositas ist zu erwarten, dass die Präsenz der hier beschriebenen Proteinfragmente von TNFSF12 zu einer signifikant verminderten Gewichtszunahme oder auch absoluten Gewichtsreduktion führt, bevorzugt verursacht durch ein signifikant vermindertes Gewicht der Fettgewebsdepots.

### Beispiel 5:

### Epitope des Fn14, welche bei Bindung durch anti-Fn14-Antikörper eine Rezeptoraktivierung zur Folge haben

Antikörper, die gegen Rezeptoren auf der Zelloberfläche gerichtet sind, binden mit definierter Spezifität und Affinität. Die Bindung eines Antikörpers an Fn14 kann dessen Aktivierung bewirken. Dazu ist die blosse Bindung des Antikörpers zwar notwendig, aber nicht hinreichend, da die Rezeptoraktivierung von der Art der Bindung des Antikörpers abhängig ist. Diese Art der Bindung durch den Antikörper muss den Effekt simulieren, welchen die Bindung eines natürlichen Liganden an den Rezeptor hat. In vielen Fällen bewirkt der natürliche Ligand eine Änderung der Form (Konformation) oder einer anderen Eigenschaft des Rezeptors, zum Beispiel seine Dimerisierung oder Multimerisierung, oder beides. Eine Aktivierung erfolgt dann, wenn diese strukturellen Änderungen eine intrinsische Aktivität des Rezeptorproteins, zum Beispiel eine Enzymaktivität, oder eine Protein-Proteinwechselwirkungsaktivität direkt oder indirekt zur Folge haben. Weil die induzierten Veränderungen des Rezeptors abhängig sind von den Epitopen auf dem Rezeptor, an den/die der Ligand oder Antikörper bindet, binden Antikörper, welche zur Aktivierung von Rezeptoren führen, an solche Epitope (hier ,aktivierendes Epitop' genannt), die bei Bindung die Rezeptoreffekte der Ligandenbindung simulieren. Das Peptid der Sequenz MDCASCRARPHSDFC stellt ein solches aktivierendes Epitop des Rezeptors Fn14 dar. Antikörper, welche an dieses Peptid binden, sind damit zur Fn14-Aktivierung in der Lage.

Um Antikörper zu identifizieren, welche an das Peptid der Sequenz MDCASCRARPHSDFC (SEQ-ID#4) binden, welches das aktivierende Epitop von Fn14 darstellt, wurden direkte Bindungsexperimente durchgeführt. Diese Experimente beruhen auf dem im Stand der Technik bekannten Prinzip des ELISA. Bei diesem ELISA Test wird die Bindung von potentiell aktivierenden Antikörpern an das Peptid der Sequenz MDCASCRARPHSDFC dadurch gemessen, dass das Peptid auf einer soliden Oberfläche (z.B. Mikrotiterplatte) immobilisiert wird, und danach die Fähigkeit von Antikörpern analysiert wird, an diese immobilisierten Peptide zu binden. Zur Kontrolle ist es bei diesen Tests notwendig, die unspezifische Bindung von Antikörpern an Kontrollpeptide zu messen, welche nicht das aktivierende Epitop repräsentieren. Antikörper, welche im Vergleich zur Bindung an das Kontrollpeptid eine deutlich verstärkte Bindung an das aktivierende Epitop zeigen (d.h. an das Peptid der Sequenz MDCASCRARPHSDFC), werden damit als 'Binder' an das aktivierende Epitop definiert. Eine weitere Kontrolle in diesen Experimenten ist die Analyse der Spezifität der Bindung an das aktivierende Epitop. Um diese zu gewährleisten, wird ebenfalls die Bindung eines weiteren unrelevanten Antikörpers sowohl an das aktivierende Epitop als auch an das Kontrollpeptid analysiert. Solche Antikörper müssen im Vergleich zu Antikörpern, welche das aktivierende Epitop erkennen, wesentlich schwächer an das aktivierende Epitop binden.

Figur 6 zeigt ein Beispiel für die Identifizierung eines Antikörpers, der spezifisch an das aktivierende Epitop von Fn14 bindet, welches durch das Peptid der Sequenz MDCASCRARPHSDFC repräsentiert wird. Hierzu wurden jeweils 0.02, 0.2, 1mg/ml des Peptides MDCASCRARPHSDFC (aktivierendes Epitop) oder eines Kontrollpeptides auf Mikrotiterplatten (MaxiSorb Platte) durch 15 h Inkubation bei 4°C in Carbonate Buffer aufgebracht (0,1 M Carbonate Buffer, 8,4 g Na HCO3 und 3,56 g Na2CO3 pro I;pH 9,5). Nach Behandlung der Platte mit Blockierpuffer (PBS pH7,4 0,5% BSA, 5 % Magermilch, 10 % FCS, 0.1 % Tween20; 2 h bei RT) und Waschpuffer (4 x 300 µl PBS + 0.05 % Tween20) zur Reduzierung unspezifischer Antikörperbindung, wurden jeweils 5 µg/ml eines Kontrollantikörpers (Maus anti GFP-Antikörper; Klon JL-8, Fa. Clontech) oder eines zu testenden anti-Fn14-Antikörpers (Maus anti human Fn14, Klon mAB-X1) in Probenpuffer (PBS + 10 % FCS, 0.1% Tween20) zugegeben und 2 Stunden bei Raumtemperatur inkubiert. Nach weiteren Waschschritten zur Entfernung ungebundener Antikörper (4 x 300 µl PBS + 0.05 % Tween20) wurde zur Detektion der spezifischen Antikörperbindung zunächst enzymgekoppelter sekundärer Antikörper (160 ng/ml ,Ziege anti Maus Antikörper gekoppelt mit HRPO (Horse raddish peroxidase), Fa. Jackson Laboratories), in Probenpuffer (PBS + 10 % FCS, 0.1% Tween20) zugegeben. Dieser wurde 1.5 Stunden bei Raumtemperatur inkubiert. Nach weiteren 4 Waschschritten zur Entfernung ungebundener sekundärer Antikörper-Enzymkonjugate (4 x 300 µl PBS + 0.05 % Tween20) wurde spezifisch gebundener Test- oder Kontrollantikörper durch Zugabe des Substrates TMB (Tetramethylbenzidin, Sigma) und darauffolgende 30 minütige Inkubation bei Raumtemperatur detektiert. Nach Abstoppen der Enzymreaktion Reaktion mit 50 µl 1 M HCl wurde die Stärke der enzymabhängigen Farbreaktion im ELISA-Reader durch Bestimmung der Absorption bei 450 nm ermittelt.
Die Resultate einer solchen ELISA Analyse ist in Figur 6 gezeigt. Es ist klar erkennbar, dass der Antikörper mAB-X1 im Vergleich zur Bindung an das Kontrollpeptid eine deutlich verstärkte Bindung an das aktivierende Epitop zeigt (d.h. an das Peptid der Sequenz MDCASCRARPHSDFC). Demnach ist mAB X1 eindeutig als Binder des aktivierenden Epitopes erkennbar. Zusätzlich zeigt die Analyse der Bindung eines Kontrollantikörpers, dass diese Bindung und der verwendete Test spezifisch ist. Denn der Kontrollantikörper zeigt geringe Bindung sowohl an das Kontrollpeptid als auch an das Peptid, welches das aktivierende Epitop representiert. Erfindungsgemässe Antikörper werden bevorzugt über dieses ELISA Verfahren identifiziert.
Um zu bestätigen, dass das Peptid der Sequenz MDCASCRARPHSDFC welches das aktivierende Epitop von Fn14 darstellt, von einem Fn14-spezifischen Antikörper erkannt wird, können zusätzlich Kompetitionsexperimente durchgeführt werden. Diese Experimente beruhen darauf, dass die spezifische Bindung an Zelloberflächen von aktivierenden anti-Fn14-Antikörpern durch Zugabe von Peptiden, welche das aktivierende Epitop repräsentieren, reduziert oder verhindert werden kann. Hingegen können Kontrollpeptide oder Peptide, welche andere Epitope repräsentieren, diese Bindung nicht beeinflussen.

Figur 5 zeigt die Resultate eines solchen Kompetitionsexperiments. Das aktivierende Epitop ist in diesem Experiment erneut durch das Peptid der Sequenz MDCASCRARPHSDFC dargestellt. Zur Durchführung des Experimentes wurde der aktivierende Antikörper (1ug/ml) mit dem Testpeptid in Konzentrationen von 0.25, 0.5, 0.75, 1 mg/ml Peptid (in 10% DMSO) in 50 µl 1xPBS + 0.5 % BSA, 0.02 % NaAzid (= PBA Puffer) versehen und 15 Stunden bei 4°C inkubiert. Daraufhin wurden 1.3 x 10⁵ He-La-Zellen , welche den Rezeptor auf der Oberfläche tragen, in 200 µl PBA mit 50 µl des Antikörper-Peptid(Protein) Ansatzes versetzt, 30 min auf Eis inkubiert um Antikörperbindung zu ermöglichen, dann pelletiert, der Überstand verworfen und das Zellpellet mit 200 µl PBA gewaschen um nicht gebundenen Antikörper zu entfernen. Daraufhin wurde AlexaFluor488 konjugierter Ziege anti Maus Antikörper (Fa. Molecular Probes) in einer Konzentration von 5 µg/ml zugegeben, für 30 Minuten auf Eis inkubiert, die Zellen anschließend 2 mal mit 200 µl PBA gewaschen und daraufhin die Anzahl der von Antikörpern gebundenen Zellen, sowie die Stärke der Bindung an diese Zellen mittels FACS-Analyse bestimmt. Dazu wurden die Zellen in einem Volumen von 200 µl im FACScalibur (Becton Dickinson) im FL1-H Kanal ohne Verwendung eines "gates" gemessen.
Die Resultate dieser FACS Analysen sind in Figur 5 gezeigt. In Figur 5A ist klar erkennbar, dass der Antikörper mAB-X1 durch das Peptid der Sequenz MDCASCRARPHSDFC, welches das aktivierende Epitop von Fn14 darstellt, in seiner Bindung an die Zellen gehemmt wird. Die Tatsache, dass diese Inhibition in einer dosisabhängigen Weise stattfindet (Figur 5B) ist ein weiterer Hinweis auf die Spezifität der Antikörperbindung und deren spezifischen Kompetition durch das Peptid der Sequenz MDCASCRARPHSDFC, welches das aktivierende Epitop repräsentiert.
Kontrollpeptide mit anderen Sequenzen aus Fn14 oder aus gänzlich anderen Proteinen zeigten in diesem Versuchsaufbau erwartungsgemäß keine signifikante Verschiebung des FACS Signals nach links - in Richtung abnehmender Markierung der HeLa Zellen durch den Antikörper - während die Zugabe der kompletten extrazellulären Domäne des Fn14 in einer Fusion mit Fc-Protein (Fa. R&D Systems; eingesetzte Menge 10 µg/ml) ebenfalls zu einer deutlichen Linksverschiebung des Signals führte (Figur 5A).

## Patentansprüche

1. Verwendung eines Fn14-aktivierenden Agenz, vorzugsweise eines Anti-Fn14-Antikörpers (Anti-Fn14-AK) zur Herstellung einer pharmazeutischen Zusammensetzung zur Vorbeugung und/oder Behandlung von Adipositas und/oder von Adipositas-assozüerter Diabetes (Typ II) und/oder Metabolischem Syndrom und/oder anderen Adipositas-assozüerten Krankheiten.

2. Verwendung eines Fn14-aktivierenden Agenz nach Anspruch 1, wobei das Agenz eine Aktivierung des Fn14-Signalwegs bewirkt.

3. Verwendung eines Fn14-aktivierenden Agenz nach einem der Ansprüche 1 oder 2, wobei die Aktivierung allein oder in Kombination mit anderen Fn14-aktivierenden Agenzien stattfindet.

4. Verwendung eines Fn14-aktivierenden Agenz nach einem der Ansprüche 1 bis 3, wobei das Agenz die Differenzierung von Präadipozyten zu reifen Adipozyten und/oder die Einlagerung von Lipidmolekülen in sich differenzierenden und/oder bereits reifen Adipozyten hemmt und/oder die Lipolyse fördert.

5. Verwendung eines Fn14-aktivierenden Agenz nach einem der Ansprüche 1 bis 4, wobei das Agenz einen agonistischen Anti-Fn14-Antikörper umfasst.

6. Verwendung eines Fn14-aktivierenden Agenz nach einem der Ansprüche 1 bis 5, wobei das Agenz gegen mindestens ein aktivierendes Epitop des Fn14 gerichtet ist und die Sequenz gemäß SEQ ID NO 4 aufweist.

7. Verwendung eines Fn14-aktivierenden Agenz nach einem der Ansprüche 1 bis 6, wobei das Agenz polyklonale und/oder monoklonale Anti-Fn14-Antikörper umfasst.

8. Verwendung eines Fn14-aktivierenden Agenz nach einem der Ansprüche 1 bis 7, wobei das Agenz funktionelle Varianten und/oder Derivate von Anti-Fn14-Antikörpern, insbesondere Moleküle mit ähnlichen Bindungseigenschaften wie chimäre, rekombinante, humanisierte oder anderweitig modifizierte Antikörper, Antikörperfragmente, Einzeldomänenantikörper und andere spezifische Bindungsproteine, umfasst.

9. Verwendung eines Fn14-aktivierenden Agenz nach einem der Ansprüche 1 bis 8, wobei das Agenz einen Agonisten, insbesondere ein Protein mit ähnlichen Bindungseigenschaften wie Antikaline, Affiline, oder ein Nicht-Protein wie Aptamere, umfasst.

10. Pharmazeutische Zusammensetzung, enthaltend ein Fn14-aktivierendes Agenz, wie in einem der Ansprüche 1 bis 9 definiert.

11. Peptid, **dadurch gekennzeichnet, dass** es mindestens eines der aktivierenden Epitope des Fn14 aufweist, ausgewählt aus der Gruppe der Sequenzen gemäß SEQ ID NO 4 oder einem Peptid, das mindestens eine fünf Aminosäuren lange Teilsequenz der Sequenz ID NO 4 enthält, und ausgenommen der vollständigen Sequenz des Fn14.

12. Antikörper, **dadurch gekennzeichnet, dass** er ein Peptid der SEQ ID NO 4 bindet.

13. Antikörper nach Anspruch 12, **dadurch gekennzeichnet, dass** er Fn14 aktiviert.

14. Verwendung eines Peptids nach Anspruch 11 zur Herstellung eines aktivierenden Anti-Fn14-Antikörpers.

15. Pharmazeutische Zusammensetzung, enthaltend ein Peptid nach Anspruch 11.

16. Verwendung eines Peptids nach Anspruch 11 zur Herstellung eines Impfstoffs.

17. Verwendung eines Peptids nach Anspruch 11 zur Herstellung eines Impfstoffes zur Vorbeugung und/oder Behandlung von Adipositas und/oder von Adipositas-assoziierter Diabetes (Typ II) und/oder Metabolischem Syndrom und/oder anderen Adipositas-assoziierten Krankheiten.

18. Verfahren zur Identifizierung eines Fn14-aktivierenden Agenz, umfassend den Schritt Inkontaktbringen des bzw. der zu testenden Agenz(ien) mit mindestens einem Stoff, enthaltend ein aktivierendes Epitop des Fn14, vorzugsweise ein Peptid nach Anspruch 11, und Nachweis einer Wechselwirkung des Agenz mit dem Epitop.

19. Verfahren nach Anspruch 18, **dadurch gekennzeichnet, dass** der Stoff von einer Zelle exprimiert wird und der Phänotyp der Zelle in Anwesenheit bzw. Abwesenheit des Agenz bestimmt wird.

20. Verfahren zur Identifizierung eines Fn14-aktivierenden Agenz, wobei durch Bindung des Agenz an mindestens ein aktivierendes Epitop des Fn14, vorzugsweise ein Peptid nach Anspruch 11, die Aktivierung des Fn14 erfolgt, und das Verfahren die folgenden Schritte umfasst:
a) Aussaat undifferenzierter Präadipozyten und Kultivieren bis zur Postkonfluenz;
b) Induzieren der Zellen und simultanes Inkubieren der zur Differenzierung angeregten Zellen mit zu testenden Agenzien;
c) Bestimmen des Differenzierungsgrads der Zellen, wobei dieses Bestimmen die Detektion des intrazellulären Lipidgehalts umfasst;
d) Identifizieren eines aktivierenden Agenz anhand der Abweichung der Test-Signalstärke gegenüber einer negativen Kontrolle;
e) Testen des in Schritt d) positiv identifizierten Agenz auf spezifische Bindung an das aktivierende Epitop des Fn14 oder eines Peptids nach Anspruch 11.

21. Verfahren nach einem der Ansprüche 18 bis 20, **dadurch gekennzeichnet, dass** die Zelle eine Säugerzelle, bevorzugt humanen Ursprungs, ist.

22. Verfahren nach einem der Ansprüche 17 bis 21, **dadurch gekennzeichnet, dass** die Zelle ein primärer Präadipozyt oder primärer Adipozyt ist oder von einem primären Präadipozyten oder primären Adipozyten abstammt.

23. Verfahren nach einem der Ansprüche 17 bis 22, **dadurch gekennzeichnet, dass** die Veränderung der biologischen Aktivität von Präadipozyten oder reifen Adipozyten, vorzugsweise 3T3-L1-Zellen oder humanen Präadipozyten oder Adipozyten, bestimmt wird.

24. Verfahren nach einem der Ansprüche 17 bis 23, **dadurch gekennzeichnet, dass** die Veränderung der biologischen Aktivität in einer Hemmung oder Verringerung der Fetteinlagerung in Zellen, vorzugsweise in Präadipozyten, sich differenzierenden Adipozyten oder reifen Adipozyten, besteht.

25. Verfahren nach einem der Ansprüche 17 bis 24, **dadurch gekennzeichnet, dass** die Fetteinlagerung mittels Bestimmung des intrazellulären eingelagerten Lipids ermittelt wird.

## Geänderte Patentansprüche

### Geänderte Patentansprüche gemäss Regel 86(2) EPÜ.

**1.** Verwendung eines agonistischen Anti-Fn14-Antikörpers zur Herstellung einer pharmazeutischen Zusammensetzung zur Vorbeugung und/oder Behandlung von Adipositas und/oder Adipositas-assoziierter Diabetes (Typ II) und/oder Metabolischem Syndrom und/oder anderen Adipositas-assoziierten Krankheiten.

**2.** Verwendung eines Anti-Fn14-Antikörper nach Anspruch 1, wobei der Antikörper eine Aktivierung des Fn14-Signalwegs bewirkt.

**3.** Verwendung eines Anti-Fn14-Antikörper nach einem der Ansprüche 1 oder 2, wobei die Aktivierung allein oder in Kombination mit anderen Fn14-aktivierenden Agenzien stattfindet.

**4.** Verwendung eines Anti-Fn14-Antikörper nach einem der Ansprüche 1 bis 3, wobei der Antikörper die Differenzierung von Präadipozyten zu reifen Adipozyten und/oder die Einlagerung von Lipidmolekülen in sich differenzierenden und/oder reifen Adipozyten hemmt und/oder die Lipolyse fördert.

**5.** Verwendung eines Anti-Fn14-Antikörper nach einem der Ansprüche 1 bis 4, wobei der Antikörper gegen mindestens ein aktivierendes Epitop des Fn14 gerichtet ist, das die Sequenz gemäß SEQ ID NO: 4 aufweist.

**6.** Verwendung eines Anti-Fn14-Antikörper nach einem der Ansprüche 1 bis 5, wobei der Antikörper polyklonale und/oder monoklonale Anti-Fn14-Antikörper umfasst.

**7.** Verwendung eines Anti-Fn14-Antikörper nach einem der Ansprüche 1 bis 6, wobei der Antikörper funktionelle Varianten von Anti-Fn14-Antikörpern, insbesondere Moleküle, die im wesentlichen die biologische Funktion oder Funktionen des Antikörpers vermitteln, wie chimäre, rekombinante oder humanisierte Antikörper und Einzeldomänenantikörper umfasst.

**8.** Verwendung eines Anti-Fn14-Antikörper nach einem der Ansprüche 1 bis 7, wobei der Antikörper einen Agonisten, insbesondere ein Protein mit ähnlichen Bindungseigenschaften wie Antikaline, Affiline, oder ein Nicht-Proteinwie Aptamere, umfasst.

**9.** Pharmazeutische Zusammensetzung, enthaltend einen Anti-Fn14-Antikörper, wie in einem der Ansprüche 1 bis 8 definiert.

**10.** Antikörper, **dadurch gekennzeichnet, dass** er ein Peptid der SEQ ID NO: 4 bindet und Fn14 aktiviert.
